(19) Europäisches Patentamt
European Patent Office
Office européen des brevets

(11) **EP 4 006 528 B1**

(12) **EUROPEAN PATENT SPECIFICATION**

(45) Date of publication and mention
of the grant of the patent:
**17.06.2026 Bulletin 2026/25**

(21) Application number: **21201755.2**

(22) Date of filing: **08.10.2021**

(51) International Patent Classification (IPC):
*G01N 21/64* (2006.01)

(52) Cooperative Patent Classification (CPC):
**G01N 21/645; G01J 3/4406;** B01L 9/06;
B01L 2300/0803; C12Q 1/686; G01J 2003/1226;
G01J 2003/1243; G01N 21/6452; G01N 2021/6419;
G01N 2021/6421; G01N 2021/6471

(54) **DETECTION DEVICE AND USE THEREOF**

DETEKTIONSVORRICHTUNG UND VERWENDUNG DAVON

DISPOSITIF DE DÉTECTION ET SON UTILISATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **27.11.2020 TW 109141915**

(43) Date of publication of application:
**01.06.2022 Bulletin 2022/22**

(73) Proprietor: **Wistron Corporation
New Taipei City 22181 (TW)**

(72) Inventor: **Chang, Yao-Tsung
22181 New Taipei City (TW)**

(74) Representative: **2K Patent Partnerschaft mbB
Hamburger Allee 26-28
60486 Frankfurt am Main (DE)**

(56) References cited:
EP-A1- 3 907 496          DE-U1- 20 321 352
US-A- 5 243 465           US-A1- 2005 151 972
US-A1- 2010 188 741       US-A1- 2016 230 210

**Description**

**BACKGROUND**

**Technical Field**

The disclosure relates to a detection device and a detection method, and more particularly to a detection device and a detection method for photofluorescence applications.

Description of Related Art

Existing detection technology for fluorescent real-time polymerase chain reaction/quantitative polymerase chain reaction (real-time PCR/qPCR) applications mainly includes a temperature control part, a detection part, and an analysis part. In the temperature control part, a temperature control device is used to generate the required thermal cycle, so that the amount of the target analyte in the test specimen is doubled after each thermal cycle, and the amount of the test specimen may be changed to $2^N$ times after N thermal cycles. In the detection part, an excitation beam whose main emission wavelength falls within a specific wavelength range is used to irradiate onto the test specimen to generate a fluorescence beam whose main emission wavelength falls within another specific wavelength range, and a light detection element is then used to receive the fluorescence beam, and detect the characteristics of the fluorescence beam. In the analysis part, an analysis software is used to monitor the temperature change and the fluorescence change of the entire polymerase chain reaction in real time, and perform quantitative analysis on the test specimen.

Generally speaking, since there are many types of fluorescent reagents on the market that are used to be added to the test specimen, and each type of fluorescent reagent has a relatively suitable excitation spectrum thereof, it is necessary to dispose a suitable optical bandpass filter (optical bandpass filter) on the optical path before the excitation beam passes through the test specimen according to the type of fluorescent reagent, so as to effectively form the required fluorescence beam when irradiating the fluorescent reagent onto the test specimen. The bandpass filter (optical bandpass filter) is a filter that allows light of a certain wavelength to pass through and prevents light of other wavelengths from passing through. In addition, since the signal of the fluorescence beam is generally quite weak and may be easily masked by the signal of other noise light, a filter module with one or several bandpass filters is usually disposed on the optical path before the light detection element receives the fluorescence beam within another specific wavelength range to filter out the signal of noise light outside the other specific wavelength range and to purify the characteristics of the fluorescence beam. In order to ensure detection accuracy, the optical density (OD) value of many bandpass filters is required to reach the OD6 level, that is, the passing rate of light passing through the cut-off wavelength of each bandpass filter must be less than or equal to $10^{-6}$.

On the other hand, when it is necessary for the existing detection device on the market to detect the test specimen with various different fluorescent reagents, multiple different fluorescence channels (that is, the overall optical path from the light source to the light detection element, including the optical path from the light source, through the optical path of the excitation beam generated, the optical path of the fluorescence beam formed by the test specimen, to the light detection element) are disposed to correspond to the requirements of various different fluorescent reagents. Also, it is necessary to dispose multiple different filter modules with different bandpass filters on each fluorescence channel to meet the requirements of forming an excitation beam with a suitable excitation spectrum and purifying the characteristics of the fluorescence beam.

As a result, according to the prior art, when the detection device is designed to simultaneously detect various test specimens with various different fluorescent reagents, the number of fluorescence channels increases, thereby increasing product costs.

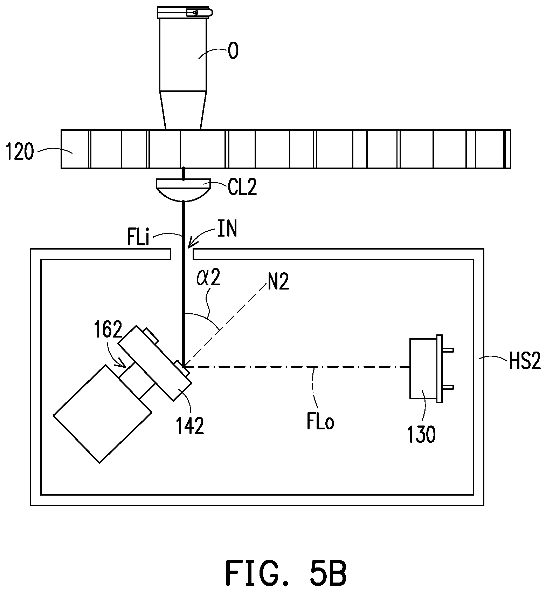

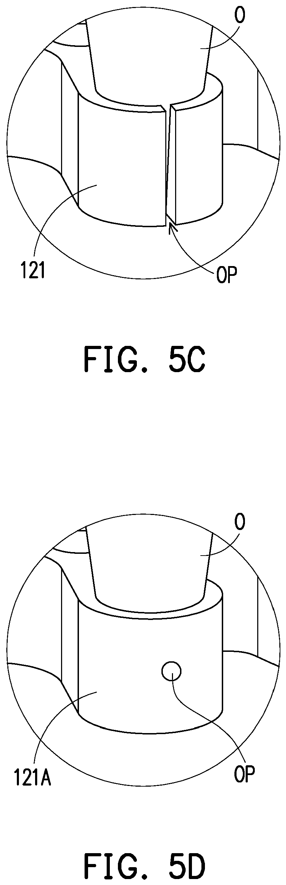

EP 3 97 496 A1 of the Applicant, which is prior art under Art. 54(3) EPC, discloses a quantitative polymerase chain reaction (qPCR) system according to the preamble of claim 1. Although with reference to Figs. 5B and 5C the existence of a second reflective optical film element is disclosed, it is not disclosed that the first reflective filter unit of the first reflective optical film element and the first reflective filter unit of the second reflective optical film element are used in pair, and that the second reflective filter unit of the first reflective optical film element and the second reflective filter unit of the second reflective optical film element are used in pair.

US 20050151972 A1 discloses another fluorometry device and method adapted to determine concentration of spectrally distinguishable species in a biological sample with a plurality of movable optical devices. The existence of a second reflective optical film element and that the first reflective filter unit of the first reflective optical film element and the first reflective filter unit of the second reflective optical film element are used in pair, and that the second reflective filter unit of the first reflective optical film element and the second reflective filter unit of the second reflective optical film element are used in pair, are not disclosed.

US 2016/230210 A1 represents the closest prior art for the invention as claimed and discloses a quantitative polymerase chain reaction (qPCR) system and corresponding detection method, wherein filter wheels are used to filter out or pass through excitation and fluorescence light of selected wavelengths. More specifically, excitation and emission filters are provided on two rotatable filter wheels. However, instead of transmissive filters reflective filters are used in the system disclosed by US 2016/230210 A1.

## SUMMARY

It is an object of the present invention to provide a quantitative polymerase chain reaction (qPCR) system and use thereof to enable detection and analysis of polymerase chain reactions reliably even when light detection signals are quite weak and multiple different fluorescent reagents or multiple different fluorescence channels are present.

This problem is solved by a quantitative polymerase chain reaction (qPCR) system as claimed in claim 1 and by the use of such a qPCR system as claimed in claim 2.

Based on the above, the detection device and the detection method of the disclosure perform fluorescence detection by the configuration of at least one reflective optical film element. According to an embodiment of the disclosure, only at least one reflective optical film element is required, while a filter module composed of bandpass filters is not required, to perform fluorescence detection, which also facilitates the update and expansion of the equipment. According to another embodiment of the disclosure, the same optical path or fluorescence channel may be used to support the detection of the test specimen with various different fluorescent reagents, thereby simplifying the optical path and reducing the complexity of the device.

## BRIEF DESCRIPTION OF THE DRAWINGS

FIG. 1 is a block diagram of a system of a detection device according to an embodiment of the disclosure, useful for understanding the invention.

FIG. 2 is a schematic diagram of an architecture of the detection device of FIG. 1.

FIG. 3A is a schematic diagram of a reflective optical film element of FIG. 2 according to an embodiment, useful for understanding the invention.

FIG. 3B to FIG. 3E are schematic diagrams illustrating the working principle of the reflective optical film element.

FIG. 4 is a schematic diagram of a flowchart of a detection method when the reflective optical film element of FIG. 3A is adopted.

FIG. 5A is a schematic diagram of an optical path of the detection device of FIG. 2 when an incident beam is an excitation beam.

FIG. 5B is a schematic diagram of an optical path of the detection device of FIG. 2 when an incident beam is a fluorescence beam.

FIG. 5C is a partially enlarged schematic diagram of a sleeve structure of FIG. 5A.

FIG. 5D is a partially enlarged schematic diagram of another sleeve structure of FIG. 5A.

FIG. 6A is a schematic diagram of the architecture of reflective optical film elements of FIG. 2 in an embodiment that is outside the subject-matter of the claims.

FIGs. 6B and FIG. 6C are schematic diagrams of architectures of different reflective optical film elements of FIG. 2 of embodiments according to the invention.

FIG. 7 is a block diagram of an application example of a fluorescent real-time polymerase chain reaction/quantitative polymerase chain reaction (real-time PCR/qPCR) system of the detection device according to an embodiment of the disclosure.

FIG. 8 is a block diagram of a system of a temperature control module in FIG. 7 according to an embodiment.

## DETAILED DESCRIPTION OF DISCLOSED EMBODIMENTS

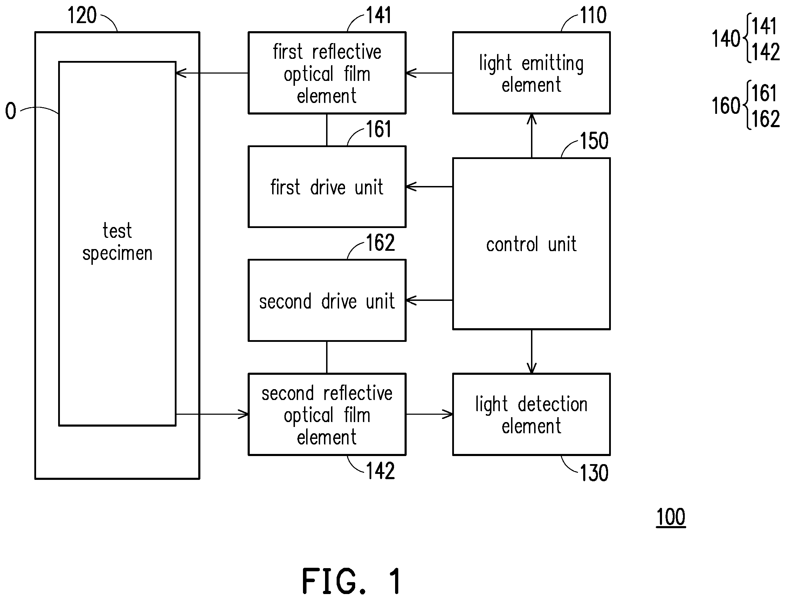

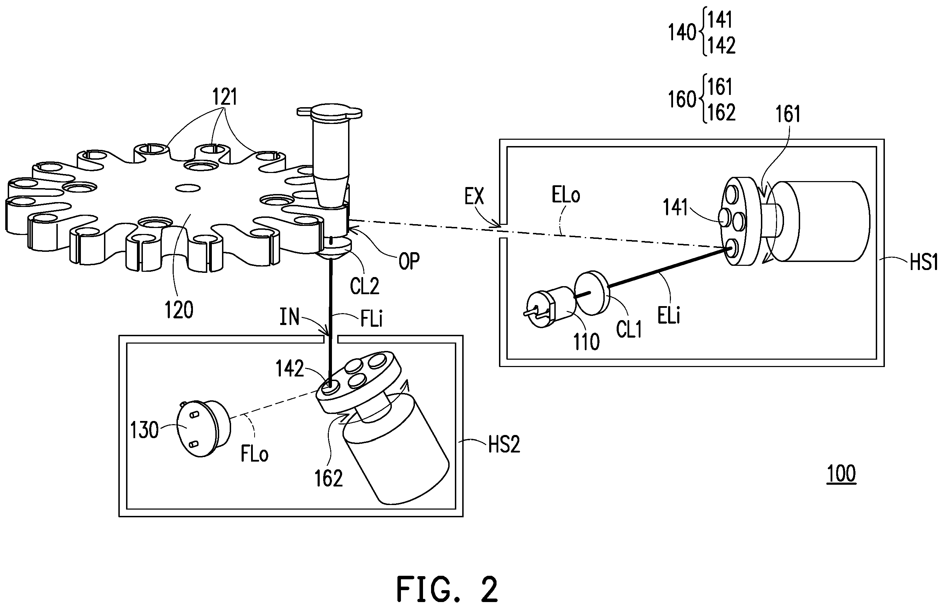

Please refer to the embodiment of the disclosure in FIG. 1 and FIG. 2. A detection device 100 of the embodiment includes a light emitting element 110 as an excitation light source, an accommodation frame 120, a light detection element 130 as a light detector, a control unit 150, at least one drive unit 160, and at least one reflective optical film element 140. The at least one drive unit 160 includes a first drive unit 161 and a second drive unit 162. The at least one optical film element 140 includes a first reflective optical film element 141 and a second reflective optical film element 142. In addition, in the embodiment, the fluorescence channel of the detection device 100 is constituted by the overall optical path from emitting element 110 to the light detection element 130, including an optical path of an excitation beam ELi from the light emitting element 110 to the first reflective optical film element 141, an optical path of an excitation beam ELo from the first reflective optical film element 141 to (a test specimen O in) the accommodation frame 120, an optical path of a fluorescence beam FLi from (the test specimen O in) the accommodation frame 120 to the second reflective optical film element 142, and an optical path of a fluorescence beam FLo from the second reflective optical film element 142 to the light detection element 130. According to an embodiment of the disclosure, the detection device 100 includes a housing (not shown) for accommodating all or part of the light emitting element 110, the accommodation frame 120, the light detection element 130, the control unit 150, the at least one drive unit 160, and the at least one reflective optical film element 140.

According to an embodiment of the disclosure, the light emitting element 110 is configured to provide the excitation beam ELi and as an excitation light source. For example, the light emitting element 110 may be a white light emitting diode, and may be configured to provide the excitation beam ELi whose emission wavelength falls between about 400 nanometers and about 700 nanometers. As another example, the light emitting element 110 may be an ultraviolet light emitting diode, and the emission wavelength range provided thereby includes at least a part of the ultraviolet light wavelength range. As still another example, the light emitting element 110 may be a light source including visible light and ultraviolet light, and the emission wavelength range provided thereby includes at least the wavelength ranges of visible light and ultraviolet light.

The accommodation frame 120 of the embodiment is configured to accommodate the test specimen O. According to an embodiment of the disclosure as shown in FIG. 2, the accommodation frame 120 has at least one sleeve structure 121. The at least one sleeve structure 121 is configured to accommodate the test specimen O. For example, as shown in FIG. 2, the test specimen O with a fluorescent reagent is placed in one of the sleeve structures 121. When the excitation spectrum suitable for the fluorescent reagent falls within the excitation wavelength range, that is, when a part of the excitation beam ELo whose main emission wavelength falls within the excitation wavelength range is irradiated onto the test specimen O, the fluorescent reagent in the test specimen O may generate the fluorescence beam FLi.

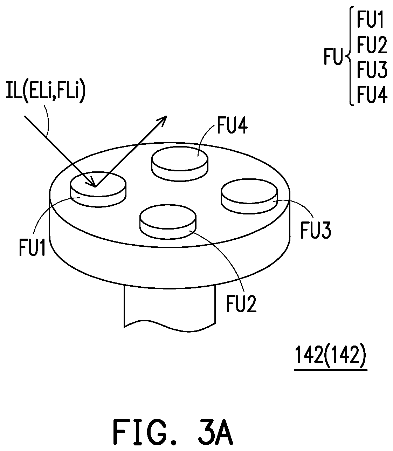

According to an embodiment of the disclosure, as shown in FIG. 2 and FIG. 3A to FIG. 3E, at least one reflective optical film element 140 may use the interference effect of light to filter out a part of the wavelength range of the incident beam IL when light enters, so as to form and reflect an outgoing beam OL whose main emission wavelength falls within a specific wavelength range. According to an embodiment of the disclosure, at least one reflective optical film element 140 includes the first reflective optical film element 141 and/or the second reflective optical film element 142. The first reflective optical film element 141 and/or the second reflective optical film element 142 may be reflective optical film elements of a microelectromechanical systems (MEMS), which can reflect the incident beam IL with a specific wavelength under the control of the control unit 150, or reflect light with a specific wavelength in the incident beam IL to become the outgoing beam OL. According to another embodiment of the disclosure, the MEMS reflective optical film element 140 may include multiple different reflective filter units FU. More specifically, the different reflective filter units FU respectively have different film structures (such as film thickness, film material, etc.), and may respectively reflect the incident beams IL with different specific wavelengths under the control of the control unit 150 to become the outgoing beams OL.

According to an embodiment of the disclosure, as shown in FIG. 3A to FIG. 3E, the first reflective optical film element 141 and the second reflective optical film element 142 respectively include multiple reflective filter units FU. The reflective filter units FU include different reflective filter units FU1, FU2, FU3, and FU4, each having a different structure, so the wavelength range of the main emission wavelength of the reflected light thereby is different. According to an embodiment of the disclosure, the reflective filter unit FU may include multiple layers of films, and the difference in film structure design (such as the difference in film thickness or the difference in film material) may enable the wavelength range of the main emission wavelength of light reflected by different reflective filter units FU to be different.

The following will be accompanied by a brief introduction of the working principle of the reflective optical film element for further explanation.

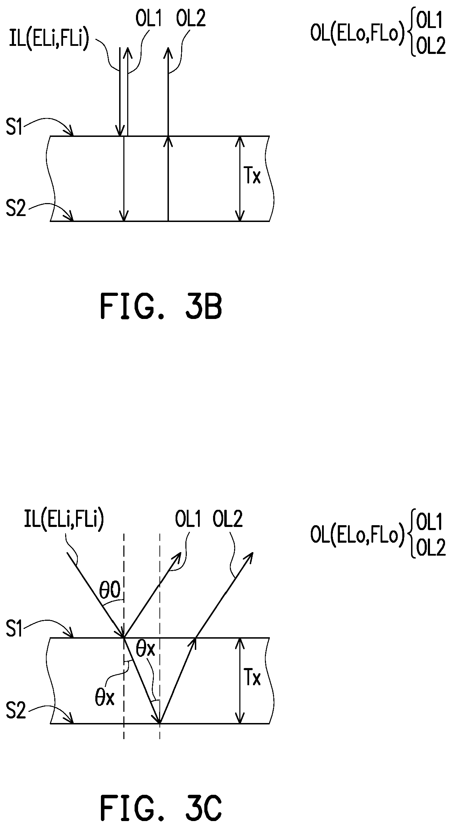

Please refer to FIG. 3B. According to the principle of film optical interference, when light enters a dense medium from a sparse medium and is reflected, the phase of the light changes by 180 degrees, but not when light enters a sparse medium from a dense medium and is reflected. Therefore, when the incident beam IL perpendicularly enters the film from a first interface S1, if the optical path difference (OPD) between the outgoing beam OL1 that exits the film after being perpendicularly reflected via the first interface S1 and the outgoing beam OL2 that enters the film and is perpendicularly reflected via a second interface S2 and then transmitted from the interface S1 to leave the film is an integral multiple of a wavelength $\lambda$ plus half of the wavelength $\lambda$, that is, optical path difference OPD = $(\lambda m - \lambda/2)$, where m is a positive integer such as 1, 2, 3, ..., the outgoing beam OL1 and the outgoing beam OL2 respectively reflected via the first interface S1 and the second interface S2 will be in phase to generate constructive interference, and the intensity of the total outgoing beam OL will increase significantly. The optical path difference is the product of twice a film thickness Tx and a film refractive index nx: optical path difference OPD = $2(Tx)*(nx) = 2(nx)(Tx)$. However, when the wavelength of the incident beam IL does not meet the above condition, the incident beam IL cannot be significantly reflected by the film. In other words, the thickness of the optical film will determine the wavelength of the outgoing beam OL that may be significantly reflected. For example, when m = 1 is selected, the optical path difference OPD = $\lambda/2$, and the constructive interference between the thickness of the optical film and the outgoing beam OL may be determined by the following relationship: $\lambda = 4(nx)(Tx)$.

Please refer to FIG. 3C. When the external incident beam IL does not enter the film perpendicularly but has an

incidence angle $\theta0$, the optical path difference between the outgoing beam OL1 and the outgoing beam OL2 respectively reflected via the first interface S1 and the second interface S2 needs to consider the relationship between parameters such as a refractive index n0 of the external medium, the refractive index nx of the film medium, and the incidence angle $\theta0$ and a refractive angle $\theta$x of the incident beam IL entering the film, consider the case where the refractive index n0 is approximately equal to 1 when the outside is vacuum or air, the following equation will be met: optical path difference OPD = 2(nx)(Tx)cos($\theta$x), where cos($\theta$x) may be obtained by using sin($\theta$x) = (n0)sin($\theta0$)/(nx) = sin($\theta0$)/(nx) (according to Snell's Law), and then consider the case of constructive interference where the optical path difference OPD = ($\lambda$m-$\lambda$/2), where m is a positive integer such as 1, 2, 3, ..., so that the constructive interference of light of the wavelength $\lambda$ can be achieved by setting a suitable film thickness Tx. For example, when m = 1 is selected, the optical path difference OPD = $\lambda$/2, the relationship between the film thickness Tx and the wavelength $\lambda$ of the outgoing beam OL may be determined through the following equation: $\lambda$=4(nx)(Tx)cos($\theta$x ), where sin($\theta$x) = sin($\theta0$)/(nx), and nx may be obtained from the medium material, so after the incidence angle $\theta0$ is determined, the reactive angle $\theta$x may be obtained, thereby obtaining cos($\theta$x). In addition, in order to obtain the outgoing beam OL with the value of the required wavelength $\lambda$, a suitable film thickness Tx may be set based on the value of the required wavelength $\lambda$ of the outgoing beam OL via the above equation.

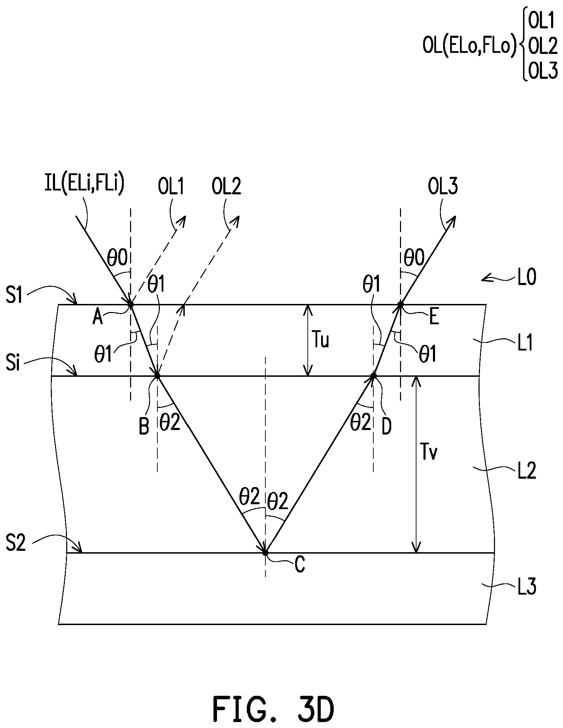

Please refer to FIG. 3D to further consider the case where there are more layers of film. The refractive indices of an external medium layer L0, a first medium layer L1, a second medium layer L2, and a third medium layer L3 from top to bottom are, for example, respectively n0, n1, n2, and n3, and n0 < n1, n2 < n1, and n2 < n3. The external medium layer L0 is, for example, the outside (such as air or vacuum), the first medium layer L1 is, for example, an optical film, the second medium layer L2 is, for example, an optical film or cavity (such as air or vacuum), and the third medium layer L3 is, for example, an optical film or mirror (such as a total reflection mirror). At this time, from top to bottom, the medium layers are respectively an optically sparse medium, an optically dense medium, an optically sparse medium, and an optically dense medium. When the external incident beam IL does not enter the film perpendicularly but has an angle, assuming that the incidence angle is the incidence angle $\theta0$, the refraction angle when passing through the first interface S1 (or an external interface S1, which is an interface between the external medium layer L0 and the first medium layer L1) at a point A is a first included angle $\theta1$, and the refraction angle when passing through an internal interface Si (an interface between the first medium layer L1 and the second medium layer L2) at a point B is a second included angle $\theta2$, and when the beam encounters the second interface S2 (or a bottom interface S2, which is an interface between the second medium layer L2 and the third medium layer (substrate) L3) at a point C, only the part reflected upward and directly passing through a point D of the internal interface Si and a point E of the first interface S1 is considered. The first medium layer L1 has a thickness Tu and the second medium layer L2 has a thickness Tv, the optical path difference OPD = 2(n1)(Tu)cos($\theta1$) + 2(n2)(Tv) cos($\theta2$) of an outgoing beam OL3 relative to the outgoing beam OL1 may be obtained, where (n0)sin($\theta0$) = (n1)sin($\theta1$) = (n2)sin($\theta2$) (according to Snell's law), and also considering that n0 < n1 and n2 < n3. Therefore, during constructive interference, the optical path difference OPD = $\lambda$m, where m is a positive integer such as 1, 2, 3, ..., so that the constructive interference of light of the wavelength $\lambda$ can be achieved by setting a suitable film thickness. The effect equation of the outgoing beam OL2 has been obtained in FIG. 3C, and the outgoing beam OL is the sum of the interference effects of the outgoing beam OL1, the outgoing beam OL2, the outgoing beam OL3, and other reflected light. When the number of layers of the optical film is further increased and multiple layers of film are stacked, the optical properties thereof may be further set to enhance the constructive interference effect of light of the wavelength $\lambda$, thereby achieving the objective of wavelength selection. By the principle of film optical interference, the main wavelength range of the desired light reflected may be selected by setting a suitable film thickness Tx of the optical film element.

More specifically, according to the principle of film optical interference, when coating a substrate, the film thickness may be adjusted to adjust the interference performance of light with different wavelengths when reflected, thereby changing the relative intensity of the reflected light with different wavelengths, so that the reflectivity of light with constructively interfered wavelengths is increased, and the reflectivity of light with destructively interfered wavelengths is decreased. Also, for a specific coated substrate (the coating structure has been fixed), in the reflected light when light is irradiated thereon, the respective reflectivity of light with different wavelengths is increased or decreased.

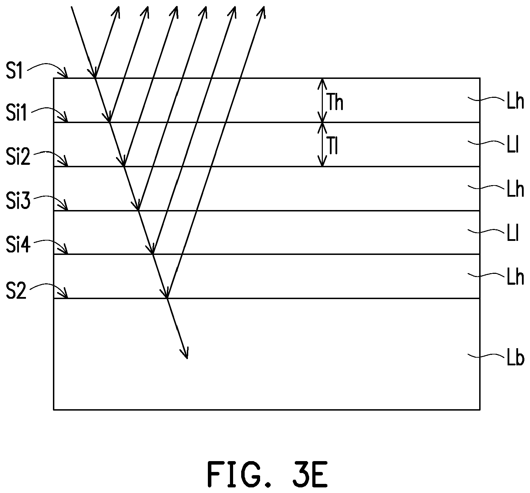

Based on the above principle, by setting multiple layers of film and considering the selection of material and the setting of film thickness, the objective of enhancing the reflection of light with a specific wavelength may be achieved to obtain a high reflection coefficient structure for light with a specific wavelength. For example, please refer to FIG. 3E. The structure is to first dispose a layer of a high refractive index material layer Lh on a substrate Lb, and then alternately stack 2 sets of low refractive index material layer Ll and high refractive index material layer Lh (Ll/Lh). The film system is stacked into S/H LH LH/A = S/H(LH)$^2$/A from bottom to top, and the thickness of each layer is a quarter of a reference wavelength $\lambda0$ (the wavelength to be reflected), which has a good effect on the constructive interference of light with the reference wavelength $\lambda0$ when reflected, where S stands for substrate Lb, H stands for high refractive index material layer Lh, L stands for low refractive index material layer Ll, and A stands for air layer. Generally speaking, when the high refractive index material layer Lh is first disposed on the substrate S, and multiple low refractive index material layers Ll and multiple high refractive index material layers Lh are then alternately stacked and coated on the substrate Lb before being adjacent to the external air medium, and the film thickness of each layer is a quarter of the reference wavelength $\lambda0$, and thus a film system structure S/H(LH)ᵖ/A may be formed. The constructive interference of reflected light may become significant to increase the overall reflectivity by passing through the first interface S1, the second interface S2, and the interfaces Si1, Si2, Si3, and Si4 therebetween. The equivalent refractive index ($n_E$) will increase, and a light reflectivity R of the reference wavelength λ0 will also increase as shown in the following equation, where $n_E$ is the equivalent refractive index, $n_S$ is the refractive index of the substrate, $n_H$ is the refractive index of the high refractive index material layer Lh, $n_L$ is the refractive index of the low refractive index material layer Ll, R is the reflectivity, and p is the number of alternately stacked sets when the low refractive index material layer L1 and the high refractive index material layer Lh (Ll/Lh) are one group, where p is a positive integer, that is, p = 1, 2, 3, .... Such reflective coating layer design is called a quarter-wave stack and may be configured to obtain high reflectivity.

$$n_E = \left(\frac{n_H}{n_L}\right)^{2p}\left(\frac{n_H^2}{n_S}\right)$$

$$R \cong 1 - \left(\frac{n_L}{n_H}\right)^{2p}\left(\frac{n_s}{n_H^2}\right)$$

According to an embodiment of the disclosure, the selection of material and the setting of film thickness may be considered and the quarter-wave stack design may be used to manufacture the internal structures of the reflective filter units FU of the first reflective optical film element 141 and the second reflective optical film element 142. According to another embodiment of the disclosure, it is also possible to consider the selection of material and the setting of film thickness, and use other high reflectivity film designs instead of the quarter-wave stack design.

Moreover, according to an embodiment of the disclosure, as shown in FIG. 1, the detection device 100 includes multiple drive units 160. The multiple drive units 160 include a first drive unit 161 and a second drive unit 162. The control unit 150 controls the first drive unit 161 and the second drive unit 162 to respectively drive operations of the first reflective optical film element 141 and the second reflective optical film element 142, so that the reflective filter unit FU of one of the two cuts into the transmission path of the excitation beam ELo, and the reflective filter unit FU of the other one cuts into the transmission path of the fluorescence beam FLo. In other words, the control unit 150 may further adjust the main emission wavelength range of the excitation beam ELo and/or the fluorescence beam FLo through controlling the movement or switching of the positions of the different reflective filter units FU1, FU2, FU3, and FU4 in the at least one reflective optical film element 140, so that the main emission wavelength of the excitation beam ELo can fall within the excitation wavelength range and/or the main emission wavelength of the fluorescence beam FLo can fall within the detection wavelength range.

More specifically, in the embodiment, the control unit 150 can select one of the reflective filter units FU1, FU2, FU3, and FU4 of the first reflective optical film element 141 based on the excitation wavelength range.

In addition, the control unit may control the first drive unit to drive one of the reflective filter units FU of the first reflective optical film element 141 to cut into the transmission path of the excitation beam ELo. For example, as shown in FIG. 2, in the embodiment, the first drive unit 161 drives operation of the first reflective optical film element 141 by rotation, but the disclosure is not limited thereto. In other embodiments, the first drive unit 161 may drive operation of the first reflective optical film element 141 by movement and/or rotation.

In this way, the control unit 150 may enable the main emission wavelength of the excitation beam ELo to fall within the excitation wavelength range through the selection of the reflective filter units FU1, FU2, FU3, and FU4 of the first reflective optical film element 141.

In this way, as long as a suitable reflective filter unit FU is selected to cut into the transmission path of the excitation beam ELo, when the excitation beam ELi passes through the first reflective optical film element 141, the required excitation beam ELo whose main emission wavelength falls within the excitation wavelength range may be formed.

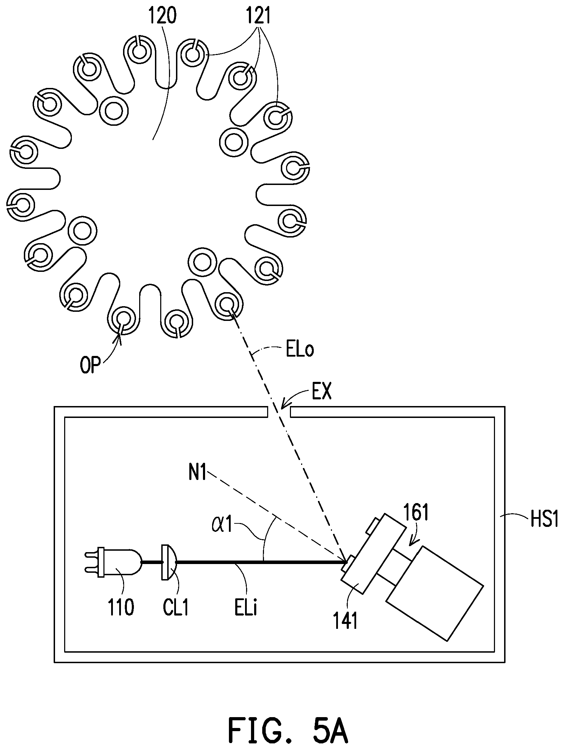

As shown in FIG. 2, FIG. 5A, and FIG. 5B, in the embodiment, the incident beam IL may be the excitation beam ELi and/or the fluorescence beam FLi, and the corresponding outgoing beam OL may respectively be the excitation beam ELo and/or the fluorescence beam FLo. Furthermore, as shown in FIG. 2 and FIG. 5A, when the incident beam IL is the excitation beam ELi, the at least one reflective optical film element 140 includes the first reflective optical film element 141. The first reflective optical film element 141 is located on the transmission path of the excitation beam ELi and is located between the light emitting element 110 and the accommodation frame 120. In other words, the incident beam IL being the excitation beam ELi is the case where the excitation beam ELi is incident on the first reflective optical film element 141. When the excitation beam ELi passes through the first reflective optical film element 141, a suitable reflective filter unit FU of the first reflective optical film element 141 is selected through controlling the switching of the positions of the different reflective filter units FU1, FU2, FU3, and FU4 of the first reflective optical film element 141 to form the excitation beam ELo whose main emission wavelength falls within the excitation wavelength range.

Moreover, as shown in FIG. 2, in the embodiment, the detection device 100 further includes a first housing HS1 to form a first darkroom for accommodating the light emitting element 110 and the first reflective optical film element 141, so as to isolate external noise light, which facilitates fluorescent detection. In addition, the first housing HS1 has an exit EX for the excitation beam ELo whose main emission wavelength falls within the excitation wavelength range to pass through. According to an embodiment of the disclosure, the detection device 100 is disposed with at least one darkroom to accommodate the test specimen O, so as to isolate external noise light, which facilitates fluorescent detection. According to another embodiment of the disclosure, the test specimen O is disposed in a darkroom and is supported by the accommodation frame 120 to isolate external noise light. However, the darkroom has an opening for the test specimen O to receive the excitation beam ELo and an opening for the test specimen O to send the fluorescence beam FLi.

In more detail, as shown in FIG. 2 and FIG. 5A, since the position of the light emitting element 110, the incident direction of the excitation beam ELi, and the position of the test specimen O are all fixed, a normal direction N1 of the first reflective optical film element 141 and a first incidence angle $\alpha 1$ between the excitation beam ELi and the normal direction N1 will also remain at a fixed value. Therefore, as long as the exit EX of the first housing HS1 is disposed as a small hole on the transmission path of the excitation beam ELo, the excitation beam ELo may pass through the exit EX to be incident on the test specimen O. According to an embodiment of the disclosure, the inner side of the first housing HS1 is made of black material or a light-absorbing material coated with black material paint to reduce the possibility of the excitation beam ELi being reflected by the internal structure of the first housing HS1 and passing through the exit EX of the first housing HS1, which may further filter out the influence of noise light. According to an embodiment of the disclosure, a normal direction of the different reflective filter units FU1, FU2, FU3, and FU4 of the first reflective optical film element 141 and a first incidence angle between the excitation beam ELi and the normal direction will remain at a fixed value as well. Furthermore, when the excitation beam ELi passes through the first reflective optical film element 141, a suitable reflective filter unit FU of the first reflective optical film element 141 is selected through controlling the switching of the positions of the different reflective filter units FU1, FU2, FU3, and FU4 of the first reflective optical film element 141 to form the excitation beam ELo whose main emission wavelength falls within the excitation wavelength range. The excitation wavelength range is the wavelength range where the characteristics of the excitation beam ELo are more prominent.

Next, as shown in FIG. 2 and FIG. 5C, according to an embodiment of the disclosure, at least one sleeve structure 121 is located on the transmission path of the excitation beam ELo. In the embodiment, each of the at least one sleeve structures 121 has an opening OP. The excitation beam ELo whose main emission wavelength falls within the excitation wavelength range will be aligned with the opening OP after passing through the exit EX of the first housing HS1, so that the opening OP may be configured to receive the excitation beam ELo. For example, the width size of the opening OP may be approximately between about 0.5 mm and 1 mm to reduce the possibility of ambient noise light passing through the opening OP and maintain the maximum value of the excitation beam ELo that the opening OP can receive. In addition, in the embodiment, the shape of the opening OP may be a slit, but the disclosure is not limited thereto. In other embodiments, the shape of the opening OP may also be a circular opening OP (such as the opening OP of a sleeve structure 121A of the embodiment of FIG. 5D), a rectangular opening OP, etc.

On the other hand, as shown in FIG. 2 and FIG. 5B, when the incident beam IL is the fluorescence beam FLi, at least one reflective optical film element 140 includes the second reflective optical film element 142. The second reflective optical film element 142 is located on the transmission path of the fluorescence beam FLi and is located between the accommodation frame 120 and the light detection element 130. In addition, there is also the opening HO under each of the at least one of the sleeve structures 121, which may be configured to emit the fluorescence beam FLi generated by a part of the excitation beam ELo whose main emission wavelength falls within the excitation wavelength range after irradiating onto the test specimen O. In other words, the incident beam IL being the fluorescence beam FLi is the case where the fluorescence beam FLi is incident on the second reflective optical film element 142. According to an embodiment of the disclosure, as shown in FIG. 2, the detection device 100 further includes a second housing HS2 to form a second darkroom for accommodating the second reflective optical film element 142 and the light detection element 130, so as to isolate external noise light, which facilitates fluorescent detection. The second housing HS2 has an inlet IN for the fluorescence beam FLi to pass through.

Moreover, similarly, as shown in FIG. 2 and FIG. 5B, since the position of the test specimen O, the incident direction of the fluorescence beam FLi, and the positions of the second reflective optical film element 142 and the light detection element 130 are all fixed, a normal direction N2 of the second reflective optical film element 142, and a second incidence angle $\alpha 2$ between the fluorescence beam FLi and the normal direction N2 will also remain at a fixed value. Therefore, as long as the inlet IN of the second housing HS2 is disposed as a small hole on the transmission path of the fluorescence beam FLi, the fluorescence beam FLi may pass through the inlet IN to be transmitted to the second reflective optical film element 142, so as to block the ambient light, thereby further filtering out the influence of noise light. According to to an embodiment of the disclosure, a normal direction of the different reflective filter units FU1, FU2, FU3, and FU4 of the second reflective optical film element 142 and a second incidence angle between the fluorescence beam FLi and the normal direction will remain at a fixed value as well. Furthermore, when the fluorescence beam FLi passes through the second reflective optical film element 142, a suitable reflective filter unit FU of the second reflective optical film element 142 is selected through controlling the switching of the positions of the different reflective filter units FU1, FU2, FU3, and FU4 of the second reflective optical film element 142 to form the fluorescence beam FLo whose main emission wavelength falls within the detection wavelength range. The detection wavelength range is the wavelength range where the characteristics of the fluorescence beam FLo are more prominent.

According to an embodiment of the disclosure, in the embodiment, the control unit 150 may also select one of the reflective filter units FU1, FU2, FU3, and FU4 as a second filter unit based on the detection wavelength range. The reflective filter unit FU1 (FU2, FU3, or FU4) of the first reflective optical film element 141 is different from the reflective filter unit FU1 (FU2, FU3, or FU4) of the second reflective optical film element 142 as the former is used for generating the excitation beam whose main emission wavelength falls within the excitation wavelength range and the latter is used for generating the fluorescence beam whose main emission wavelength falls within the detection wavelength range.

In addition, the control unit may control the second drive unit 162 to drive one of the reflective filter units FU of the second reflective optical film element 142 to cut into the transmission path of the fluorescence beam FLo. For example, as shown in FIG. 2, in the embodiment, the second drive unit 162 drives operation of the second reflective optical film element 142 by rotation, but the disclosure is not limited thereto. In other embodiments, the second drive unit 162 may drive operation of the second reflective optical film element 142 by movement and/or rotation.

In this way, the control unit 150 may also obtain the required fluorescence beam FLo whose main emission wavelength falls within the detection wavelength range through the selection of different reflective filter units FU (that is, the reflective filter units FU1, FU2, FU3, and FU4) of the second reflective optical film element 142.

Although FIG. 2, FIG. 5A, and FIG. 5B include the first darkroom and the second darkroom, according to another embodiment of the disclosure, the detection device 100 is disposed with at least one darkroom on one of the light emitting element 110 and the light detection element 130, so that the two are blocked from each other and only the optical path of the fluorescence channel is communicated, so as to reduce the noise light received by the light detection element 130, which facilitates fluorescence detection. According to another embodiment of the disclosure, the fluorescence channel of the detection device 100 from the light emitting element 110 to the light detection element 130 is disposed in at least one dark room to isolate external noise light. According to still another embodiment of the disclosure, the fluorescence channel of the detection device 100 from the light emitting element 110 to the light detection element 130 passes through at least two darkrooms, so as to reduce external noise light.

Moreover, as shown in FIG. 2, the light detection element 130 is located on the transmission path of the fluorescence beam FLo and may be configured to receive the fluorescence beam FLo and as a light detector. For example, the light detection element 130 is a photoelectric sensor capable of detecting light intensity, which may be a photodiode. Specifically, the light detection element 130 is configured to receive a part of the fluorescence beam FLo whose main emission wavelength falls within the detection wavelength range.

On the other hand, as shown in FIG. 1, in the embodiment, the detection device 100 further includes the control unit 150. For example, the control unit 150 may be a microcontroller or a central processing unit, which includes a memory, an input controller, and an output controller. According to an embodiment of the disclosure, the control unit 150 may execute a program to control the setting of the emission wavelength range of the light emitting element 110 and control the switching-on and switching-off of the light emitting element 110. According to another embodiment of the disclosure, the control unit 150 may control the light detection element 130 to adjust the detected light intensity. For example, when the light detection element 130 has different sensing intensities for light with different wavelengths, the sensing intensity may be offset through the control unit 150.

According to another embodiment of the disclosure, the control unit 150 may control the excitation beam ELi or the fluorescence beam FLi to pass through the reflective filter unit FU of the first reflective optical film element 141 and/or the second reflective optical film element 142 of at least one optical film element 140. More specifically, the control unit 150 can select the reflective filter unit FU of at least one optical film element 140 to cut into the transmission path of the excitation beam ELo and/or the fluorescence beam FLo, so as to further adjust the main emission wavelength range of the excitation beam ELo and/or the fluorescence beam Flo, so that the main emission wavelength of the excitation beam ELo can fall within the excitation wavelength range and/or the main emission wavelength of the fluorescence beam FLo can fall within the detection wavelength range.

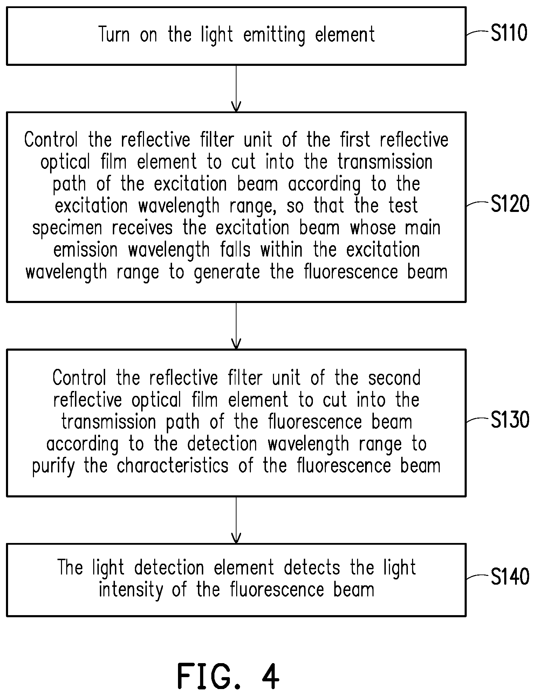

The following will further explain how the control unit 150 executes the detection method of FIG. 4. Please refer to FIG. 4. In the embodiment, the detection method of FIG. 4 may be executed by, for example, using the detection device 100 in FIG. 1 and FIG. 2.

First, Step S110 is executed. The control unit 150 turns on the light emitting element 110. Specifically, as shown in FIG. 2, in Step S110, the excitation beam ELi provided by the light emitting element 110 may be collimated into a parallel beam through a collimating lens CL1. According to another embodiment of the disclosure, the collimating lens CL1 may be omitted.

Then, Step S120 is executed. The control unit 150 controls the reflective filter unit FU of the first reflective optical film element to cut into the transmission path of the excitation beam according to the excitation wavelength range, so that the test specimen receives the excitation beam ELo whose main emission wavelength falls within the excitation wavelength range to generate the fluorescence beam FLi. More specifically, as shown in FIG. 2 and FIG. 5B, when the excitation beam ELi is incident on the first reflective optical film element 141, the control unit 150 selects the reflective filter unit FU of the first reflective optical film element 141 to cut into the transmission path of the excitation beam, so as to reflect the excitation beam ELo whose main emission wavelength falls within the excitation wavelength range, which is received by the test specimen O to generate the fluorescence beam FLi.

According to an embodiment of the disclosure, the setting value of the excitation wavelength range may be approximately between 400 nm and 700 nm to meet the specification requirements of various fluorescent reagents. Table 1 shows the rated absorption excitation wavelengths (peak values of excitation wavelengths) of several commercially available fluorescent reagents and the corresponding rated fluorescence wavelengths (peak values of fluorescence wavelengths) generated thereby as follows:

Table 1 Summary of excitation wavelengths and fluorescence wavelengths of various fluorescent reagents

| Type | Peak value of excitation wavelength | Peak value of fluorescence wavelength |
|---|---|---|
| 6-FAM, FITC | 494 nm | 520 nm |
| FITC-dT | 494 nm | 520 nm |
| TET | 521 nm | 541 nm |
| JOE | 520 nm | 548 nm |
| Yakima Yellow | 526 nm | 548 nm |
| HEX | 535 nm | 553 nm |
| Cy3 | 547 nm | 563 nm |
| TAMRA | 555 nm | 576 nm |
| ROX | 575 nm | 602 nm |
| Texas Red | 595 nm | 615 nm |
| Cy5 | 646 nm | 662 nm |

Fluorescent reagents, which includes green light (6-FAM) with an excitation wavelength of 494 nm corresponding to a fluorescence wavelength of 520 nm, yellow light (Cy3) with an excitation wavelength of 547 nm corresponding to a fluorescence wavelength of 563 nm, orange light (ROX) with an excitation wavelength of 575 nm corresponding to a fluorescence wavelength of 602 nm, red light (Cy5) with an excitation wavelength of 646 nm corresponding to a fluorescence wavelength of 662 nm, etc., are listed in Table 1. The so-called rated excitation wavelength (the peak value of the excitation wavelength) of a fluorescent reagent refers to that the fluorescent reagent reactant has a fluorescence generating effect on the excitation light with a certain excitation wavelength range, but has the best fluorescence generating effect on the rated excitation wavelength in the excitation wavelength range. In other words, the fluorescent reagent reactant has the effect of generating fluorescence for the excitation light near the rated excitation wavelength (that is, the excitation wavelength range), but the effect of generating fluorescence at the rated excitation wavelength is the best. Moreover, when the excitation wavelength range is the wavelength range covered by the excitation wavelength range, the fluorescent reagent may be applied to the detection device 100, and the reflective filter unit FU of the first reflective optical film element 141 is selected by the detection device 100 through the control unit 150, thereby enabling the excitation beam ELo to excite the test specimen O, so as to generate a better fluorescence generating effect.

Similarly, the so-called rated fluorescence wavelength (the peak value of the fluorescence wavelength) of a fluorescent reagent refers to that the fluorescence generated by the fluorescent reagent reactant to the excitation light will fall within a certain fluorescence wavelength range, but when the reactant is irradiated by the light with the rated excitation wavelength, the fluorescence wavelength generated thereby will fall near the rated fluorescence wavelength (that is, the fluorescence wavelength range), but the rated fluorescence wavelength has the best fluorescent effect. In addition, when the fluorescence wavelength range is the wavelength range covered by the detection wavelength range, the fluorescent reagent may be applied to the detection device 100, and a suitable reflective filter unit FU of the second reflective optical film element 142 is selected by the detection device 100 through the control unit 150, thereby purifying the color purity of the fluorescence beam FLo to purify the characteristics of the fluorescence beam FLo.

According to an embodiment of the disclosure, the excitation wavelength range may be within a range of 40 nm including the rated excitation wavelength. According to another embodiment of the disclosure, the excitation wavelength range may be within a range of 20 nm including the rated excitation wavelength. According to yet another embodiment of the disclosure, the excitation wavelength range may be within a range of 10 nm including the rated excitation wavelength. According to still another embodiment of the disclosure, the excitation wavelength range may be within a range of 6 nm including the rated excitation wavelength. In addition, according to an embodiment of the disclosure, the excitation wavelength range is centered on the rated excitation wavelength and is increased or decreased by a specific wavelength, such as an increase or decrease of 20 nm, an increase or decrease of 10 nm, an increase or decrease of 5 nm, or an increase or decrease of 3 nm.

Moreover, when the excitation beam ELo is required to have a specific main emission wavelength, a suitable reflective filter unit FU of the first reflective optical film element 141 may be selected. For example, when the main emission wavelength (that is, the excitation wavelength) of the excitation beam ELo is about 494 nm, the reflective filter unit FU1 corresponding to 494 nm may be selected to achieve the objective. As another example, when the main emission wavelength (that is, the excitation wavelength) of the excitation beam ELo is about 547 nm, the reflective filter unit FU2 corresponding to 547 nm may be selected to achieve the objective, and so on. In this way, as long as different reflective filter units FU are selected to cut into the transmission path of the excitation beam, the required excitation beam ELo may be obtained. Furthermore, according to an embodiment of the disclosure, the switching of the position of the reflective filter unit FU of the first reflective optical film element 141 may be controlled to switch between the excitation wavelengths of two different colors of light, such as switching from green to yellow, from yellow to orange, or from orange to red. Moreover, as the number of different reflective filter units FU increases, the types of excitation wavelengths of different colors of light that can be obtained also increase. For example, when the number of different reflective filter units FU is three, the control unit may control the switching of the positions of the different reflective filter units FU to switch between the excitation wavelengths of three different colors of light, such as green, yellow, and orange, or yellow, orange, and red. According to another embodiment of the disclosure, when the number of different reflective filter units FU is four or more, the control unit may control the switching of the positions of the different reflective filter units FU to switch between the excitation wavelengths of four or more different colors of light, such as green, yellow, orange, and red.

In this way, the control unit 150 may select the reflective filter unit FU according to the suitable wavelength range of the excitation beam ELo required by the type of fluorescent reagent in the test specimen O to effectively form the required excitation beam ELi without the need to dispose various different filter modules composed of bandpass filters and/or various different fluorescence channels as in the prior art. Also, the control unit 150 only needs to adjust the reflective filter unit FU of the first reflective optical film element 141 to support the detection of various different fluorescent reagents, which facilitates the update and expansion of the equipment. In addition, since the excitation beams ELo required by different test specimens O may share the same optical path or fluorescence channel when detecting various different fluorescent reagents, the optical path can also be simplified and the complexity of production, assembly, maintenance, and adjustment can be reduced to reduce product costs and improve production quality.

Similarly, with the different types of fluorescent reagents, the range of the main emission spectrum of the fluorescence beam FLi generated by the test specimen O will also be different. Therefore, the detection device 100 may also execute Step S130 through the configuration of the second reflective optical film element 142 located between the accommodation frame 120 and the light detection element 130. According to the detection wavelength range, the reflective filter unit FU of the second reflective optical film element is controlled to cut into the transmission path of the fluorescence beam to filter out signals of noise light outside the specific wavelength range and purify the color purity of the fluorescence beam, so as to purify the characteristics of the fluorescence beam to improve detection accuracy.

According to an embodiment of the disclosure, similar to the above principle of controlling the first reflective optical film element 141, as shown in FIG. 2 and FIG. 5B, when the fluorescence beam FLi enters the second reflective optical film element 142, the control unit 150 selects the reflective filter unit FU of the second reflective optical film element 142 to correspond to the value of the detection wavelength range. The reflective filter units FUs of the first reflective optical film element (141) and of the second reflective optical film element (142) are used in pairs.

According to an embodiment of the disclosure, the value of the detection wavelength range may be approximately between 450 nm and 730 nm to meet the specification requirements of various fluorescent reagents. Please refer to the above examples of the rated absorption excitation wavelengths of commercially available fluorescent reagents and the corresponding rated fluorescence wavelengths generated thereby. In addition, when the wavelength range (that is, a certain fluorescence wavelength range) near the rated fluorescence wavelength corresponding to different fluorescent reagent specifications is the wavelength range covered by the detection wavelength range, the fluorescent reagent may be applied to the detection device 100, and the reflective filter unit FU of the second reflective optical film element 142 is selected by the detection device 100 through the control unit 150, thereby purifying the color purity of the fluorescence beam FLo to purify the characteristics of the fluorescence beam FLo.

Furthermore, according to an embodiment of the disclosure, the fluorescence wavelength range may be within a range of 40 nm including the rated fluorescence wavelength. According to another embodiment of the disclosure, the fluorescence wavelength range may be within a range of 20 nm including the rated fluorescence wavelength. According to yet another embodiment of the disclosure, the fluorescence wavelength range may be within a range of 10 nm including the rated fluorescence wavelength. According to still another embodiment of the disclosure, the fluorescence wavelength range may be within a range of 6 nm including the rated fluorescence wavelength. In addition, according to an embodiment of the disclosure, the fluorescence wavelength range is centered on the rated fluorescence wavelength and is increased or decreased by a specific wavelength range, such as an increase or decrease of 20 nm, an increase or decrease of 10 nm, an increase or decrease of 5 nm, or an increase or decrease of 3 nm.

Moreover, when the fluorescence beam FLo is required to have a specific main emission wavelength, a suitable reflective filter unit FU of the second reflective optical film element 142 may be selected. For example, when the main emission wavelength (that is, the detection wavelength) of the fluorescence beam FLo is required to be about 520 nm, the reflective filter unit FU1 corresponding to 520 nm may be selected to achieve the objective. For another example, when the main emission wavelength (that is, the detection wavelength) of the fluorescence beam FLo is required to be about 563 nm, the reflective filter unit FU2 corresponding to 563 nm may be selected to achieve the objective, and so on. In this way, as long as a suitable reflective filter unit FU is selected to cut into the transmission path of the fluorescence beam FLo, the required fluorescence beam FLo may be obtained. Furthermore, the absorption excitation wavelengths of commercially available fluorescent reagents and the corresponding fluorescence wavelengths generated thereby are as described above. According to an embodiment of the disclosure, the reflective filter unit FU of the second reflective optical film element 142 may be selected to switch between the fluorescence wavelengths of two, three, four, or more different colors of light, similar to the case of selecting the reflective filter unit FU of the first reflective optical film element 141.

Next, please refer to FIG. 2 and FIG. 4. The control unit 150 may execute Step S140 to detect the light intensity of the fluorescence beam FLo whose main emission wavelength falls within the detection wavelength range, and convert the fluorescence beam FLo into an electrical signal for subsequent analysis.

According to an embodiment of the disclosure, the control unit 150 may set the reflective filter unit FU according to the wavelength range of the main emission spectrum of the type of fluorescent reagent in the test specimen O to filter out the signal of noise light outside the specific wavelength range and purify the characteristics of the fluorescence beam FLo without the need to dispose a filter module composed of bandpass filters. Also, the control unit 150 only needs to select the reflective filter unit FU of the second reflective optical film element 142 to cut into the transmission path of the fluorescence beam FLo to support the detection of various different fluorescent reagents, which facilitates the update and expansion of the equipment. In addition, since the fluorescence beams FLo formed by different test specimens O may share the same optical path when detecting various different fluorescent reagents, the optical path can also be simplified and the complexity of production and assembly can be reduced to reduce product costs and improve production quality.

For example, in the embodiment, when the suitable excitation spectrum of a first fluorescent reagent of a first test specimen falls within a first excitation wavelength range (the suitable excitation wavelength range of the first fluorescent reagent) and the main emission wavelength of the fluorescence beam emitted thereby falls within a first detection wavelength range (the suitable detection wavelength range of the first fluorescent reagent), the reflective filter unit FU1 of the first reflective optical film element 141 may be set such that at least a part of the main emission wavelength falls within the first excitation wavelength range, and the reflective filter unit FU1 of the second reflective optical film element 142 may be set such that at least a part of the main emission wavelength falls within the first detection wavelength range, and the reflective filter unit FU1 of the first reflective optical film element 141 and the reflective filter unit FU1 of the second reflective optical film element 142 respectively cut into the transmission path of the excitation beam ELo and/or the fluorescence beam FLo to be used in pair. When the suitable excitation spectrum of a second fluorescent reagent of a second test specimen falls within a second excitation wavelength range (the suitable excitation wavelength range of the second fluorescent reagent) and the main emission wavelength of the fluorescence beam emitted thereby falls within a second detection wavelength range (the suitable detection wavelength range of the second fluorescent reagent), the reflective filter unit FU2 of the first reflective optical film element 141 may be set such that at least a part of the main emission wavelength falls within the second excitation wavelength range, and the reflective filter unit FU2 of the second reflective optical film element 142 may be set such that at least a part of the main emission wavelength falls within the second detection wavelength range, and the reflective filter unit FU2 of the first reflective optical film element 141 and the reflective filter unit FU2 of the second reflective optical film element 142 respectively cut into the transmission path of the excitation beam ELo and/or the fluorescence beam FLo to be used in pair. The first fluorescent reagent and the second fluorescent reagent are different fluorescent reagents.

According to another embodiment of the disclosure, the first excitation wavelength range (the suitable excitation wavelength range of the first fluorescent reagent) and the second excitation wavelength range (the suitable excitation wavelength range of the second fluorescent reagent), and the respective corresponding first detection wavelength range (the suitable detection wavelength range of the first fluorescent reagent) and second detection wavelength range (the suitable detection wavelength range of the second fluorescent reagent) may respectively set the reflective filter units FU thereof.

In this way, the control unit 150 of the detection device 100 may also select the suitable reflective filter unit FU of the first reflective optical film element 141 and/or the reflective filter unit FU of the second reflective optical film element 142 to cut into the transmission path of the excitation beam ELo and/or the fluorescence beam FLo according to the suitable wavelength range (that is, the excitation wavelength range) of the excitation beam ELo required by the type of fluorescent reagent in the test specimen O or the range of the main emission spectrum (that is, the detection wavelength range) of the fluorescence beam FLo generated by the test specimen O, thereby enabling the excitation beam ELi or the fluorescence beam FLi to pass through the suitable reflective filter units FU, so as to form the required excitation beam ELo and/or fluorescence beam FLo. In this way, the detection device 100 may support the detection of various different fluorescent reagents.

In addition, it is worth noting that the first reflective optical film element 141 and the second reflective optical film element 142 are two different elements, so the shapes, sizes, arrangements, film thicknesses, numbers, and optical characteristics of the reflective filter units FU1, FU2, FU3, and FU4 of the first reflective optical film element 141 and the reflective filter units FU1, FU2, FU3, and FU4 of the second reflective optical film element 142 do not need to be the same. Furthermore, in the embodiment, the number of different reflective filter units FU of the first reflective optical film element 141 and the second reflective optical film element 142 of at least one reflective optical film element 140 is four as an example, but the disclosure is not limited thereto. According to another embodiment of the disclosure, the number of different reflective filter units FU in the first reflective optical film element 141 and the second reflective optical film element 142 of the at least one reflective optical film element 140 may be two, three, five, six, or more. In other embodiments, the reflective filter units FU of the at least one reflective optical film element 140 and the number of reflective filter units FU therein may be determined according to the number of types of fluorescent reagents, and the selection of the reflective filter units FU is simultaneously adjusted according to the characteristics of the types of fluorescent reagents to meet the requirements of the actual product.

In addition, although the detection device 100 according to the embodiment of FIG. 1 and FIG. 2 includes the light emitting element 110, the accommodation frame 120, the light detection element 130, and the at least one reflective optical film element 140, and the at least one reflective optical film element 140 includes the first reflective optical film element 141 and the second reflective optical film element 142, according to another embodiment of the disclosure, the at least one reflective optical film element 140 only includes one of the first reflective optical film element 141 and the second reflective optical film element 142. According to an embodiment of the disclosure, in an implementation where the at least one reflective optical film element 140 only includes the first reflective optical film element 141 but not the second reflective optical film element 142 (not shown), detection may be correctly performed as long as the quality of the fluorescence beam of the test specimen O is good enough, the optical path design of the fluorescence channel is good enough, or there are other reasons that enable the light detected by the light detection element 130 to meet the specifications, which also has the effect of improving the shortcomings of the prior art. At this time, the one or more drive units 160 include the first drive unit 161 for driving the first reflective optical film element 141. Also, the fluorescence channel is constituted by various sections of the optical paths between the light emitting element 110, the first reflective optical film element 141, the accommodation frame 120 (or the test specimen O), and the light detection element 130. According to another embodiment of the disclosure, in an implementation where the at least one reflective optical film element 140 only includes the first reflective optical film element 141 but not the second reflective optical film element 142 (not shown), a conventional bandpass filter (not shown) may also be included to replace the second reflective optical film element 142 in the embodiment of FIG. 1 and FIG. 2 to filter out light outside the detection wavelength range, which also has the effect of improving the shortcomings of the prior art. At this time, the fluorescence channel is constituted by various sections of the optical paths between the light emitting element 110, the first reflective optical film element 141, the accommodation frame 120 (or the test specimen O), the bandpass filter (not shown), and the light detection element 130. According to an embodiment of the disclosure, in an implementation where the at least one reflective optical film element 140 only includes the second reflective optical film element 142 but not the first reflective optical film element 141, detection may be correctly performed as long as the quality of the excitation beam of the light emitting element 110 is good enough, the quality of the fluorescence beam of the test specimen O is good enough, the optical path design of the fluorescence channel is good enough, or there are other reasons that enable the light detected by the light detection element 130 to meet the specifications, which also has the effect of improving the shortcomings of the prior art. At this time, the one or more drive units 160 include the second drive unit 162 for driving the second reflective optical film element 142. Also, the fluorescence channel is constituted by various sections of the optical paths between the light emitting element 110, the accommodation frame 120 (or the test specimen O), the second reflective optical film element 142, and the light detection element 130. According to another embodiment of the disclosure, in an implementation where the at least one reflective optical film element 140 only includes the second reflective optical film element 142 but not the first reflective optical film element 141 (not shown), a conventional bandpass filter (not shown) may also be included to replace the first reflective optical film element 141 in the embodiment of FIG. 1 and FIG. 2 to filter out light outside the excitation wavelength range, which also has the effect of improving the shortcomings of the prior art. At this time, the fluorescence channel constituted by various sections of the optical paths between the light emitting element 110, the bandpass filter (not shown), the accommodation frame 120 (or the test specimen O), the second reflective optical film element 142, and the light detection element 130.

It is worth noting that, in the above embodiments, although the operation manner of the first reflective optical film element 141 and the second reflective optical film element 142 of the reflective optical film element 140 is exemplified by rotation, the disclosure is not limited thereto. In other embodiments, the operation manner of the reflective optical film element 140 may also be by movement or simultaneous movement and rotation, and corresponding adjustments are made according to the optical requirements thereof. After referring to the disclosure, persons skilled in the art may make appropriate changes to the operation manner of the reflective optical film element 140, so that the detection device may still achieve the above effects and advantages, which should still fall within the scope of the disclosure. Hereinafter, some other embodiments will be exemplified as illustration.

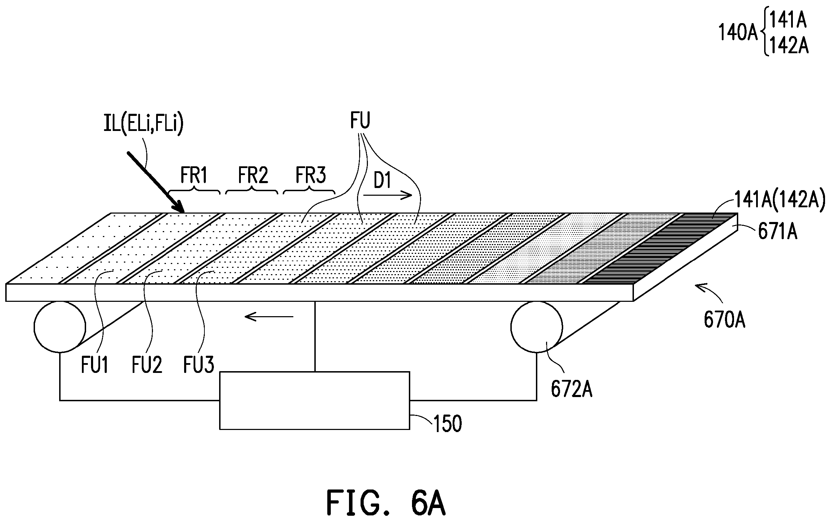

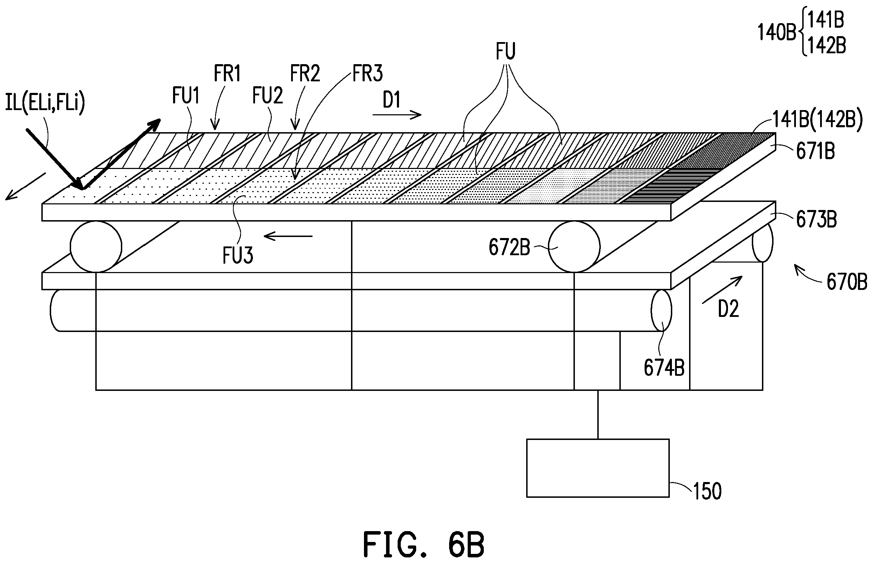

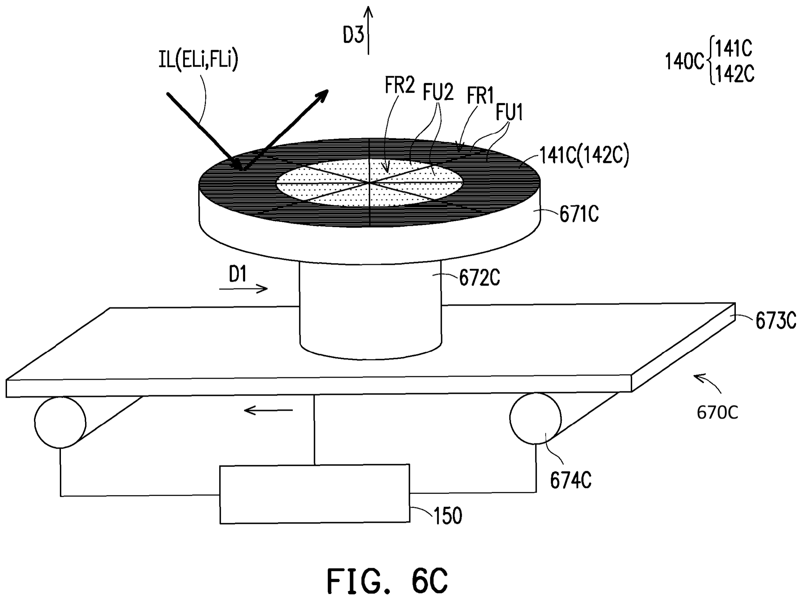

FIG. 6A is a schematic diagram of the architecture of reflective optical film elements of FIG. 2 in an embodiment that is outside the subject-matter of the claims. FIGs. 6B and FIG. 6C are schematic diagrams of architectures of different reflective optical film elements 140 (141, and 142) and drive units 160 (161, and 162) of FIG. 2 of embodiments according to the invention. Please refer to FIG. 6A to FIG. 6C, reflective optical film elements 140A, 140B, and 140C are similar to the reflective optical film element 140 of FIG. 1, and the main differences are as follows. As shown in FIG. 6A to FIG. 6C, the reflective optical film elements 140A, 140B, and 140C include multiple filter regions FR1, FR2, and FR3. Each of the filter regions FR1, FR2, and FR3 correspondingly includes one or more reflective filter units FU1, FU2, and FU3. The reflective filter units of the same FU in the same filter region are the same, but the reflective filter units in different filter regions are different from each other. That is, the filter region FR1 includes one or more reflective filter units FU1, the filter region FR2 includes one or more reflective filter units FU2, and the reflective filter unit FU1 located in the filter region FR1 is different from the reflective filter unit FU2 of the filter region FR2, and so on.

According to an embodiment that is outside the subject-matter of the claims, as shown in FIG. 6A, the first drive unit 161 and/or the second drive unit 162 is a uniaxial moving device 670A, which has a carrier 671A for carrying the reflective optical film element 140A, and has a mechanical power providing mechanism 672A (such as a motor/motors and/or a gear/gears/gear transmission, and/or other mechanical power providing mechanisms) coupled to the carrier 671A and driving the carrier 671A to translate in a first axial direction D1, so as to drive the reflective optical film element 140A to translate in the first axial direction D1.

Furthermore, as shown in FIG. 6A, the control unit 150 is electrically connected to the uniaxial moving device 670A, controls the mechanical power providing mechanism 672A, and further controls the movement of the carrier 671A to drive the first reflective optical film element 141A and the second reflective optical film element 142A of the reflective optical film element 140A, so as to enable the reflective optical film element 140A to translate along the first axial direction D1 while maintaining the same incidence angle for the incident beam IL, so that one of the filter units FU cuts into the transmission path of the excitation beam ELo, and/or one of the filter regions FR cuts into the transmission path of the fluorescence beam FLo.

According to a first embodiment of the invention, as shown in FIG. 6B, the first drive unit 161 and/or the second drive unit 162 is a biaxial moving device 670B, which has a carrier 671B for carrying the reflective optical film element 140B, has a mechanical power providing mechanism 672B (such as a motor/motors and/or a gear/gears/gear transmission, and/or other mechanical power providing mechanisms) coupled to the carrier 671B and driving the carrier 671B to translate in the first axial direction D1, so as to drive the reflective optical film element 140B to translate in the first axial direction D1, has another carrier 673B for carrying the mechanical power providing mechanism 672B, and has another mechanical power providing mechanism 674B (such as a motor/motors and/or a gear/gears/gear transmission, and/or other mechanical power providing mechanisms) coupled to the other carrier 673B and driving the other carrier 673B to translate in the second axial direction D2, so as to drive the reflective optical film element 140B to translate in the second axial direction D2. Furthermore, as shown in FIG. 6B, the control unit 150 is electrically connected to the biaxial moving device 670B, controls the mechanical power providing mechanism 672B and the other mechanical power providing mechanism 674B, and further controls the movements of the carrier 671B and the other carrier 673B to drive the first reflective optical film element 141B and the second reflective optical film element 142B of the reflective optical film element 140B, so as to enable the reflective optical film element 140B to translate along the first axial direction D1 and/or the second axial direction D2 while maintaining the same incidence angle for the incident beam IL, so that one of the reflective filter units FU cuts into the transmission path of the excitation beam ELo, and/or one of the filter regions FR cuts into the transmission path of the fluorescence beam FLo.

According to another embodiment of the invention, as shown in FIG. 6C, the first drive unit 161 and/or the second drive unit 162 is a moving turntable device 670C, which has a carrier 671C for carrying the reflective optical film element 140C, has a mechanical power providing mechanism 672C (such as a motor/motors and/or a gear/gears/gear transmission, and/or other mechanical power providing mechanisms) coupled with the carrier 671C and driving the carrier 671C to rotate relative to a third axial direction D3, so as to drive the reflective optical film element 140B to rotate relative to the third axial direction D3, has another carrier 673C for carrying the mechanical power providing mechanism 672C, and has another mechanical power providing mechanism 674C (such as a motor and/or a gear/gears, and/or other mechanical power providing mechanisms) coupled to the other carrier 673C and driving the other carrier 673C to translate in the first axial direction D1, so as to drive the reflective optical film element 140B to translate in the first axial direction D1, so that the multiple reflective filter units FU of the reflective optical film element 140C may be respectively located at different positions in the radial direction or the circumferential direction of the moving turntable device 670C. In this way, when the moving turntable device 670C rotates or moves, different reflective filter units FU located at different positions in the circumferential direction or the radial direction may respectively cut into the transmission path of the incident beam IL.

Furthermore, as shown in FIG. 6C, the control unit 150 is also electrically connected to the moving turntable device 670C, controls the mechanical power providing mechanism 672C and the other mechanical power providing mechanism 674C, and further controls the rotation of the carrier 671C and the translation of the other carrier 673C to drive the first reflective optical film element 141C and the second reflective optical film element 142C of the reflective optical film element 140C, so as to enable the reflective optical film element 140C to rotate along the third axial direction D3 and/or translate along the first axial direction D1 while maintaining the same incidence angle for the incident beam IL, so that one of the reflective filter units FU cuts into the transmission path of the excitation beam ELo, and/or one of the filter regions FR cuts into the transmission path of the fluorescence beam FLo.

In this way, when the reflective optical film elements 140A, 140B, and 140C are applied to the detection device 100 shown in FIG. 1 and FIG. 2, the detection device 100 may also control the first drive unit 161 and/or the second drive unit 162 through the control unit 150 to respectively drive the first reflective optical film element 141 and/or the second reflective optical film element 142, so that one of the reflective filter units FU of the first reflective optical film element 141 cuts into the transmission path of the excitation beam ELi/ELo, and/or one of the reflective filter units FU of the second reflective optical film element 142 cuts into the transmission path of the fluorescence beam FLi/FLo. That is, the control unit 150 may further adjust the range of the main emission wavelengths of the excitation beam ELo and the fluorescence beam FLo through controlling the switching of the positions of the different reflective filter units FU in the at least one reflective optical film element 140, so as to enable the main emission wavelength of the excitation beam ELo to fall within the excitation wavelength range and/or the main emission wavelength of the fluorescence beam FLo to fall within the detection wavelength range, which facilitates the update and expansion of the equipment, thereby achieving similar effects and advantages of the detection device 100 as described above, which will not be repeated here. According to another embodiment of the disclosure, incidence angles for the incident beam IL on the filter units FU of the reflective optical film element 140A/140B/140C maintain the same while cutting into the transmission paths of the excitation beam ELi/ELo, and/or of the fluorescence beam FLi/FLo.

In summary, the detection device of the disclosure may support the detection of various different fluorescent reagents by the configuration of the reflective optical film element without the need to dispose a filter module composed of bandpass filters, which facilitates the update and expansion of the equipment. In addition, since when the detection of various different fluorescent reagents is performed, the excitation beams (or the fluorescence beams formed) required by different test specimens may share the same optical path and/or fluorescence channel, the optical path can be simplified and the complexity of production and assembly can be reduced to reduce product costs.

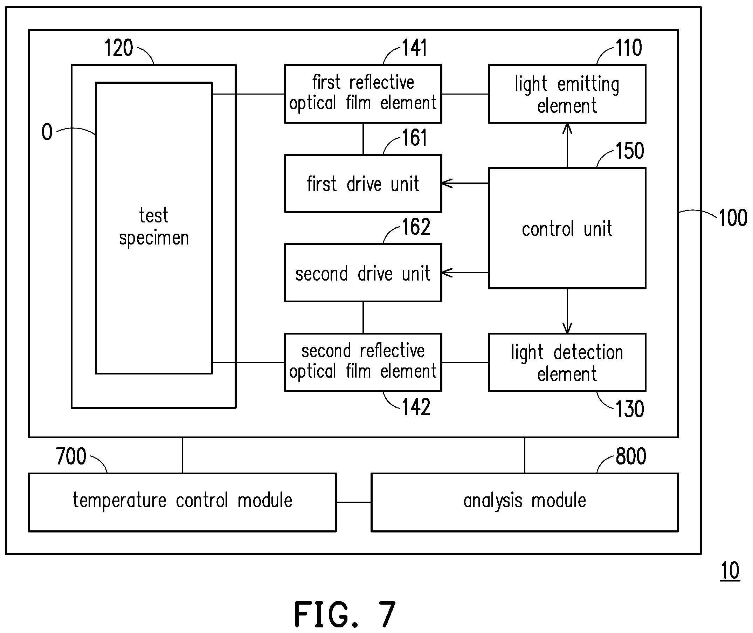

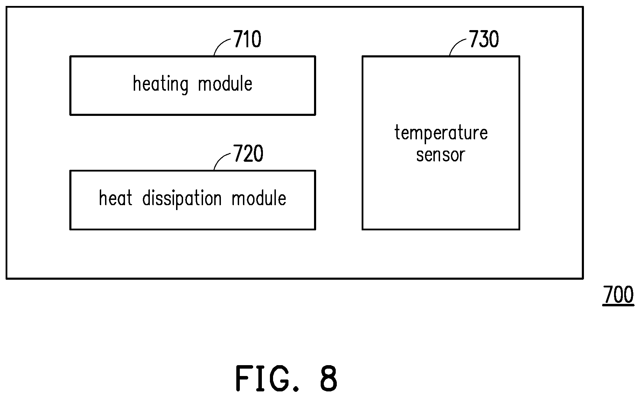

FIG. 7 is a block diagram of an application example of a fluorescent real-time polymerase chain reaction/quantitative polymerase chain reaction (real-time PCR/qPCR) system of the detection device according to the disclosure. FIG. 8 is a block diagram of a system of a temperature control module in FIG. 7 according to an embodiment. Please refer to FIG. 7 and FIG. 8, which illustrate a block diagram of an application example of a fluorescent real-time PCR/qPCR system of the detection device according to the disclosure. The detection device 100 of the disclosure may be applied to a fluorescent real-time PCR/qPCR system 10. The fluorescent real-time PCR/qPCR system 10 includes the detection device 100, a temperature control module 700, and an analysis module 800. According to an application example of the disclosure, as shown in FIG. 7, the temperature control module 700 has a heating module 710 and a heat dissipation module 720. The required thermal cycle is generated under the control of the control unit and the test specimen is temperature controlled, so that the amount of a target analyte in the test specimen is doubled after each thermal cycle, and the amount of the target analyte becomes $2^N$ times after N thermal cycles. According to an embodiment of the disclosure, the temperature control module 700 has a temperature sensor 730 for sensing a temperature of the system, such as the temperature of the accommodation frame 120, the test specimen, etc., and the test specimen may be temperature controlled through the accommodation frame 120. According to an embodiment of the disclosure, the temperature sensor 730 is connected to the accommodation frame 120 of the detection device 100 to sense the temperature of the accommodation frame 120. According to another embodiment of the disclosure, the temperature sensor 730 is connected to the sleeve structure 121 of the accommodation frame 120 to sense the temperature of the sleeve structure 121. The detection device 100 includes a light emitting element 110, a light detection element 130, at least one reflective optical film element 140 (141 and/or 142) disposed on the fluorescence channel between the light emitting element 110 and the light detection element 130, a control unit 150, and at least one first drive unit 160 (161 and/or 162) for driving the at least one reflective optical film element 140 to control the wavelength range of the reflected light. The details, operation methods, and various implementations thereof have been described in the foregoing, which will not be repeated here. The analysis module 800 monitors, records, quantitatively and/or qualitatively analyzes in real time the temperature change and the fluorescence change of the test specimen during the entire polymerase chain reaction process under the control of the control unit 150. According to an embodiment of the disclosure, the analysis module 800 uses an analysis software for analysis. According to an embodiment of the disclosure, the analysis module 800 analyzes the signal obtained by the light detection element 130.

Claims

1.  A quantitative polymerase chain reaction (qPCR) system (10), comprising:

    a detection device (100), a temperature control module (700), and an analysis module (800), wherein the detection device (100) comprises:

    an excitation light source (110);
    a light detector (130);
    a first reflective optical film element (141), disposed on a fluorescence channel between the excitation light source (110) and the light detector (130);
    a first drive unit (161), configured to drive operation of the first reflective optical film element (141); and
    a control unit (150), configured to control the first drive unit (161); wherein
    the temperature control module (700) is configured to control a temperature of the qPCR system (10), and the analysis module (800) is configured to analyze a signal from the light detector (130), wherein
    the first reflective optical film element (141) has a first reflective filter unit (FU, FU1, FU2, FU3, FU4) and a second reflective filter unit (FU, FU1, FU2, FU3, FU4), respectively configured to obtain light within a first wavelength range and light within a second wavelength range;
    the detection device (100) further comprises a second reflective optical film element (142), an accommodation frame (120) for accommodating a test specimen, and a second drive unit (162);
    the first reflective optical film element (141) is disposed on the fluorescence channel between the excitation light source (110) and the accommodation frame (120);
    the second reflective optical film element (142) is disposed on the fluorescence channel between the accommodation frame (120) and the light detector (130);
    the second drive unit (162) is configured to drive operation of the second reflective optical film element (142); and
    the control unit (150) is further configured to control the second drive unit (162), wherein
    the second reflective optical film element (142) has a first reflective filter unit (FU, FU1, FU2, FU3, FU4) and a second reflective filter unit (FU, FU1, FU2, FU3, FU4), the first and the second reflective filter unit (FU, FU1, FU2, FU3, FU4) of the first reflective optical film element (141) are respectively configured to obtain light within a first excitation wavelength range and light within a second excitation wavelength range when irradiated by an excitation beam (ELo), and the first and the second reflective filter unit (FU, FU1, FU2, FU3, FU4) of the second reflective optical film element (142) are respectively configured to obtain light within a first fluorescence wavelength range and a second fluorescence wavelength range when irradiated by a fluorescence beam (FLo),
    wherein the first reflective filter unit (FU, FU1, FU2, FU3, FU4) of the first reflective optical film element (141) and the first reflective filter unit (FU, FU1, FU2, FU3, FU4) of the second reflective optical film element (142) are used in pairs, and
    the second reflective filter unit (FU, FU1, FU2, FU3, FU4) of the first reflective optical film element (141) and the second reflective filter unit (FU, FU1, FU2, FU3, FU4) of the second reflective optical film element (142) are used in pairs,

    with one of the following arrangements:

    1) the first drive unit (161) and/or the second drive unit (162) is a biaxial moving device (670B), which has a carrier (671B) for carrying the respective first or second reflective optical film element (140B), has a mechanical power providing mechanism (672B) coupled to the carrier (671B) and driving the carrier (671B) to translate in a first axial direction (D1), so as to drive the reflective optical film element (140B) to translate in the first axial direction (D1), has another carrier (673B) for carrying the mechanical power providing mechanism (672B), and has another mechanical power providing mechanism (674B) coupled to the other carrier (673B) and driving the other carrier (673B) to translate in a second axial direction (D2) different from the first axial direction (D1), so as to drive the reflective optical film element (140B) to translate in the second axial direction (D2), so as to enable the respective first or second reflective optical film element (140B) to translate along the first axial direction (D1) and/or the second axial direction (D2), so that one of the reflective filter units (FU, FU1, FU2, FU3, FU4) of the first reflective optical film element cuts into a transmission path of the excitation beam (ELo), and/or one of the reflective filter units (FU, FU1, FU2, FU3, FU4) of the second reflective optical film element cuts into a transmission path of the fluorescence beam (FLo),
    or

2) the first drive unit (161) and/or the second drive unit (162) is a moving turntable device (670C), which has a carrier (671C) for carrying the the respective first or second reflective optical film element (140C), has a mechanical power providing mechanism (672C) coupled with the carrier (671C) and driving the carrier (671C) to rotate relative to a third axial direction (D3), so as to drive the respective first or second reflective optical film element (140B) to rotate relative to the third axial direction (D3), has another carrier (673C) for carrying the mechanical power providing mechanism (672C), and has another mechanical power providing mechanism (674C) coupled to the other carrier (673C) and driving the other carrier (673C) to translate in the first axial direction (D1), so as to drive the respective first or second reflective optical film element (140B) to translate in the first axial direction (DI), so that the multiple reflective filter units (FU) of the the respective first or second reflective optical film element (140C) may be respectively located at different positions in the radial direction or the circumferential direction of the moving turntable device (670C) so that, when the moving turntable device (670C) rotates or moves, one of the reflective filter units (FU, FU1, FU2, FU3, FU4) of the first reflective optical film element cuts into a transmission path of the excitation beam (ELo), and/or one of the the reflective filter units (FU, FU1, FU2, FU3, FU4) of the second reflective optical film element cuts into a transmission path of the fluorescence beam (FLo).

2. Use of a quantitative polymerase chain reaction (qPCR) system (10) as claimed in claim 1 in a detection method for monitoring, recording, and quantitatively and/or qualitatively analyzing in real time the temperature change and the fluorescence change of a test specimen during a polymerase chain reaction process under the control of the control unit (150), the detection method comprising:

controlling, using the control unit (150), the first drive unit (161) and/or the second drive for performing one of the following steps 1) or 2):

1) driving the carrier (671B) to translate in the first axial direction (D1), so as to drive the reflective optical film element (140B) to translate in the first axial direction (D1), and driving the other carrier (673B) to translate in the second axial direction (D2) different from the first axial direction (D1), so as to drive the reflective optical film element (140B) to translate in the second axial direction (D2), so as to enable the respective first or second reflective optical film element (140B) to translate along the first axial direction (D1) and/or the second axial direction (D2), so that one of the reflective filter units (FU, FU1, FU2, FU3, FU4) of the first reflective optical film element cuts into a transmission path of the excitation beam (ELo), and/or one of the reflective filter units (FU, FU1, FU2, FU3, FU4) of the second reflective optical film element cuts into a transmission path of the fluorescence beam (FLo); or

2) driving the carrier (671C) to rotate relative to a third axial direction (D3), so as to drive the respective first or second reflective optical film element (140B) to rotate relative to the third axial direction (D3), and driving the other carrier (673C) to translate in the first axial direction (D1), so as to drive the respective first or second reflective optical film element (140B) to translate in the first axial direction (DI), so that the multiple reflective filter units (FU) of the the respective first or second reflective optical film element (140C) may be respectively located at different positions in the radial direction or the circumferential direction of the moving turntable device (670C) so that, when the moving turntable device (670C) rotates or moves, one of the reflective filter units (FU, FU1, FU2, FU3, FU4) of the first reflective optical film element cuts into a transmission path of the excitation beam (ELo), and/or one of the the reflective filter units (FU, FU1, FU2, FU3, FU4) of the second reflective optical film element cuts into a transmission path of the fluorescence beam (FLo).

**Patentansprüche**

1. System (10) für eine quantitative Polymerasekettenreaktion (qPCR), umfassend:

eine Detektionsvorrichtung (100), ein Temperaturregelungsmodul (700) und ein Analysemodul (800), wobei die Detektionsvorrichtung (100) umfasst:

eine Anregungslichtquelle (110);
einen Lichtdetektor (130);
ein erstes reflektierendes optisches Folienelement (141), das in einem Fluoreszenzkanal zwischen der Anregungslichtquelle (110) und dem Lichtdetektor (130) angeordnet ist;
eine erste Antriebseinheit (161), die so konfiguriert ist, dass sie den Betrieb des ersten reflektierenden optischen Folienelements (141) steuert; und
eine Steuereinheit (150), die so konfiguriert ist, dass sie die erste Antriebseinheit (161) steuert; wobei
das Temperaturregelungsmodul (700) so konfiguriert ist, dass es eine Temperatur des qPCR-Systems (10) regelt, und

das Analysemodul (800) so konfiguriert ist, dass es ein Signal von dem Lichtdetektor (130) analysiert, wobei das erste reflektierende optische Folienelement (141) eine erste reflektierende Filtereinheit (FU, FU1, FU2, FU3, FU4) und eine zweite reflektierende Filtereinheit (FU, FU1, FU2, FU3, FU4) aufweist, die jeweils so konfiguriert sind, dass sie Licht innerhalb eines ersten Wellenlängenbereichs und Licht innerhalb eines zweiten Wellenlängenbereichs erzeugen;

die Detektionsvorrichtung (100) ferner ein zweites reflektierendes optisches Folienelement (142), einen Aufnahmerahmen (120) zur Aufnahme einer Probe und eine zweite Antriebseinheit (162) umfasst;

das erste reflektierende optische Folienelement (141) auf bzw. in dem Fluoreszenzkanal zwischen der Anregungslichtquelle (110) und dem Aufnahmerahmen (120) angeordnet ist;

das zweite reflektierende optische Folienelement (142) ist auf dem Fluoreszenzkanal zwischen dem Aufnahmerahmen (120) und dem Lichtdetektor (130) angeordnet;

die zweite Antriebseinheit (162) ist so konfiguriert, dass sie den Betrieb des zweiten reflektierenden optischen Folienelements (142) steuert; und

die Steuereinheit (150) ferner so konfiguriert ist, dass sie die zweite Antriebseinheit (162) steuert, wobei das zweite reflektierende optische Folienelement (142) eine erste reflektierende Filtereinheit (FU, FU1, FU2, FU3, FU4) und eine zweite reflektierende Filtereinheit (FU, FU1, FU2, FU3, FU4) aufweist, wobei die erste und die zweite reflektierende Filtereinheit (FU, FU1, FU2, FU3, FU4) des ersten reflektierenden optischen Folienelements (141) jeweils so konfiguriert sind, dass sie bei Bestrahlung durch einen Anregungsstrahl (ELo) Licht innerhalb eines ersten Anregungswellenlängenbereichs und Licht innerhalb eines zweiten Anregungswellenlängenbereichs erzeugen, und die erste und die zweite reflektierende Filtereinheit (FU, FU1, FU2, FU3, FU4) des zweiten reflektierenden optischen Folienelements (142) jeweils so konfiguriert sind, dass sie Licht innerhalb eines ersten Fluoreszenzwellenlängenbereichs und eines zweiten Fluoreszenzwellenlängenbereichs erzeugen, wenn sie von einem Fluoreszenzstrahl (FLo) bestrahlt werden,

wobei die erste reflektierende Filtereinheit (FU, FU1, FU2, FU3, FU4) des ersten reflektierenden optischen Folienelements (141) und die erste reflektierende Filtereinheit (FU, FU1, FU2, FU3, FU4) des zweiten reflektierenden optischen Folienelements (142) paarweise verwendet werden, und

die zweite reflektierende Filtereinheit (FU, FU1, FU2, FU3, FU4) des ersten reflektierenden optischen Folienelements (141) und die zweite reflektierende Filtereinheit (FU, FU1, FU2, FU3, FU4) des zweiten reflektierenden optischen Folienelements (142) paarweise verwendet werden,

mit einer der folgenden Anordnungen:

1) die erste Antriebseinheit (161) und/oder die zweite Antriebseinheit (162) ist eine zweiachsige Bewegungsvorrichtung (670B), die einen Träger (671B) zum Halten des jeweiligen ersten oder zweiten reflektierenden optischen Folienelements (140B) aufweist, einen mechanischen Kraftübertragungsmechanismus (672B) aufweist, der mit dem Träger (671B) gekoppelt ist und den Träger (671B) antreibt, sich in einer ersten axialen Richtung (D1) zu verschieben, um das reflektierende optische Folienelement (140B) anzusteuern, sich in der ersten axialen Richtung (D1) zu verschieben, einen weiteren Träger (673B) zum Halten des mechanischen Antriebsmechanismus (672B) aufweist und einen weiteren mechanischen Antriebsmechanismus (674B) aufweist, der mit dem anderen Träger (673B) gekoppelt ist und den anderen Träger (673B) ansteuert, sich in einer zweiten axialen Richtung (D2) zu verschieben, die sich von der ersten axialen Richtung (D1) unterscheidet, um das reflektierende optische Folienelement (140B) anzusteuern, sich in der zweiten axialen Richtung (D2) zu bewegen, sodass das jeweilige erste oder zweite reflektierende optische Folienelement (140B) entlang der ersten axialen Richtung (D1) und/oder der zweiten axialen Richtung (D2) verstellt werden kann, so dass eine der reflektierenden Filtereinheiten (FU, FU1, FU2, FU3, FU4) des ersten reflektierenden optischen Folienelements in einen Übertragungsweg des Anregungsstrahls (ELo) hinein verstellt ist und/oder eine der reflektierenden Filtereinheiten (FU, FU1, FU2, FU3, FU4) des zweiten reflektierenden optischen Folienelements in einen Übertragungsweg des Fluoreszenzstrahls (FLo) hinein verstellt ist, oder

2) die erste Antriebseinheit (161) und/oder die zweite Antriebseinheit (162) eine bewegliche Drehtischvorrichtung (670C) ist, die einen Träger (671C) zum Halten des jeweiligen ersten oder zweiten reflektierenden optischen Folienelements (140C) aufweist, einen mechanischen Antriebsmechanismus (672C), der mit dem Träger (671C) gekoppelt ist und den Träger (671C) ansteuert, sich relativ zu einer dritten axialen Richtung (D3) zu drehen, um das jeweilige erste oder zweite reflektierende optische Folienelement (140B) dazu anzutreiben, sich relativ zur dritten axialen Richtung (D3) zu drehen, einen weiteren Träger (673C) zum Halten des mechanischen Antriebsmechanismus (672C) sowie einen weiteren mechanischen Antriebsmechanismus (674C), der mit dem anderen Träger (673C) gekoppelt ist und den anderen Träger (673C) dazu ansteuert, dass dieser in der ersten axialen Richtung (D1) verstellt wird, um das jeweilige erste oder zweite reflektierende optische Folienelement (140B) so anzusteuern, dass es in der ersten axialen Richtung (D1) verstellt wird, sodass die mehreren reflektierenden Filtereinheiten (FU) des jeweiligen ersten oder zweiten reflektierenden optischen Folienelements (140C) jeweils an unterschiedlichen Positionen in der radialen Richtung oder der Umfangsrichtung der beweglichen Drehtischvorrichtung (670C) angeordnet sein können, so dass, wenn sich die bewegliche Drehtischvorrichtung (670C) dreht oder bewegt, eine der reflektierenden Filtereinheiten (FU, FU1, FU2, FU3, FU4) des ersten reflektierenden optischen Folienelements in einen Übertragungsweg des Anregungsstrahls (ELo) hinein verstellt ist und/oder eine der reflektierenden Filtereinheiten (FU, FU1, FU2, FU3, FU4) des zweiten reflektierenden optischen Folienelements in einen Übertragungsweg des Fluoreszenzstrahls (FLo) hinein verstellt ist.

2. Verwendung eines quantitativen Polymerasekettenreaktionssystems (qPCR-Systems) (10) nach Anspruch 1 in einem Detektionsverfahren zur Überwachung, Aufzeichnung und quantitativen und/oder qualitativen Analyse der Temperaturänderung und der Fluoreszenzänderung einer Testprobe während eines Polymerasekettenreaktionsprozesses in Echtzeit unter der Steuerung der Steuereinheit (150), wobei das Detektionsverfahren umfasst: Ansteuern der ersten Antriebseinheit (161) und/oder der zweiten Antriebseinheit unter Verwendung der Steuereinheit (150), um einen der folgenden Schritte 1) oder 2) auszuführen:

1) Ansteuern des Trägers (671B), sodass dieser in der ersten axialen Richtung (D1) verstellt wird, um das reflektierende optische Folienelement (140B) anzusteuern, sodass dieses in der ersten axialen Richtung (D1) verstellt wird, und Ansteuern des anderen Trägers (673B), sodass dieser in der zweiten axialen Richtung (D2) verstellt wird, die sich von der ersten axialen Richtung (D1), sodasss das reflektierende optische Folienelement (140B) in der zweiten axialen Richtung (D2) verstellt wird, damit das jeweilige erste oder zweite reflektierende optische Folienelement (140B) entlang der ersten axialen Richtung (D1) und/oder der zweiten axialen Richtung (D2) verstellt werden kann, sodass eine der reflektierenden Filtereinheiten (FU, FU1, FU2, FU3, FU4) des ersten reflektierenden optischen Folienelements in einen Übertragungsweg des Anregungsstrahls (ELo) eintritt und/oder eine der reflektierenden Filtereinheiten (FU, FU1, FU2, FU3, FU4) des zweiten reflektierenden optischen Folienelements in einen Übertragungsweg des Fluoreszenzstrahls (FLo) eintritt; oder

2) Ansteuern des Trägers (671C), sodass dieser relativ zu einer dritten axialen Richtung (D3) verstellt wird, um das jeweilige erste oder zweite reflektierende optische Folienelement (140B) relativ zur dritten axialen Richtung (D3) zu drehen, und Ansteuern des anderen Trägers (673C) zur Verstellung in der ersten axialen Richtung (D1), sodass das jeweilige erste oder zweite reflektierende optische Folienelement (140B) zur Verstellung in der ersten axialen Richtung (D1) angesteuert wird, sodass die mehreren reflektierenden Filtereinheiten (FU) des jeweiligen ersten oder zweiten reflektierenden optischen Folienelements (140C) jeweils an unterschiedlichen Positionen in der radialen Richtung oder der Umfangsrichtung der beweglichen Drehtischvorrichtung (670C) angeordnet werden können, so dass, wenn sich die bewegliche Drehtischvorrichtung (670C) dreht oder bewegt, eine der reflektierenden Filtereinheiten (FU, FU1, FU2, FU3, FU4) des ersten reflektierenden optischen Folienelements in einen Übertragungsweg des Anregungsstrahls (ELo) eintritt und/oder eine der reflektierenden Filtereinheiten (FU, FU1, FU2, FU3, FU4) des zweiten reflektierenden optischen Folienelements in einen Übertragungsweg des Fluoreszenzstrahls (FLo) eintritt.

## Revendications

1. Un système de réaction en chaîne par polymérase quantitative (qPCR) (10), comprenant :

un dispositif de détection (100), un module de contrôle de température (700) et un module d'analyse (800), dans lequel
le dispositif de détection (100) comprend :

une source de lumière d'excitation (110) ;
un détecteur de lumière (130) ;
un premier élément de film optique réfléchissant (141), disposé sur un canal de fluorescence entre la source de lumière d'excitation (110) et le détecteur de lumière (130) ;
une première unité de commande (161), configurée pour commander le fonctionnement du premier élément de film optique réfléchissant (141) ; et
une unité de commande (150), configurée pour commander la première unité d'entraînement (161) ; dans lequel

le module de contrôle de température (700) est configuré pour contrôler une température du système qPCR (10), et

le module d'analyse (800) est configuré pour analyser un signal provenant du détecteur de lumière (130), dans lequel

le premier élément de film optique réfléchissant (141) comporte une première unité de filtre réfléchissant (FU, FU1, FU2, FU3, FU4) et une deuxième unité de filtre réfléchissant (FU, FU1, FU2, FU3, FU4), configurées respectivement pour obtenir de la lumière dans une première gamme de longueurs d'onde et de la lumière dans une deuxième gamme de longueurs d'onde ;

le dispositif de détection (100) comprend en outre un deuxième élément de film optique réfléchissant (142), un cadre de logement (120) destiné à loger un échantillon à tester, et une deuxième unité d'entraînement (162) ;

le premier élément de film optique réfléchissant (141) est disposé sur le canal de fluorescence entre la source de lumière d'excitation (110) et le cadre de logement (120) ;

le deuxième élément de film optique réfléchissant (142) est disposé sur le canal de fluorescence entre le cadre de logement (120) et le détecteur de lumière (130) ;

la deuxième unité d'entraînement (162) est configurée pour commander le fonctionnement du deuxième élément de film optique réfléchissant (142) ; et

l'unité de commande (150) est en outre configurée pour commander la deuxième unité d'entraînement (162), dans laquelle

le deuxième élément de film optique réfléchissant (142) comporte une première unité de filtre réfléchissant (FU, FU1, FU2, FU3, FU4) et une deuxième unité de filtre réfléchissant (FU, FU1, FU2, FU3, FU4), les première et deuxième unités de filtre réfléchissant (FU, FU1, FU2, FU3, FU4) du premier élément de film optique réfléchissant (141) sont respectivement configurées pour obtenir de la lumière dans une première plage de longueurs d'onde d'excitation et de la lumière dans une deuxième plage de longueurs d'onde d'excitation lorsqu'elles sont irradiées par un faisceau d'excitation (ELo), et les première et deuxième unités de filtre réfléchissant (FU, FU1, FU2, FU3, FU4) du deuxième élément de film optique réfléchissant (142) sont respectivement configurées pour obtenir de la lumière dans une première plage de longueurs d'onde de fluorescence et une deuxième plage de longueurs d'onde de fluorescence lorsqu'elles sont irradiées par un faisceau de fluorescence (FLo),

dans lequel la première unité de filtre réfléchissant (FU, FU1, FU2, FU3, FU4) du premier élément de film optique réfléchissant (141) et la première unité de filtre réfléchissante (FU, FU1, FU2, FU3, FU4) du deuxième élément de film optique réfléchissant (142) sont utilisées par paires, et

la deuxième unité de filtre réfléchissante (FU, FU1, FU2, FU3, FU4) du premier élément de film optique réfléchissant (141) et la deuxième unité de filtre réfléchissant (FU, FU1, FU2, FU3, FU4) du deuxième élément de film optique réfléchissant (142) sont utilisées par paires,

avec l'une des configurations suivantes :

1) la première unité d'entraînement (161) et/ou la deuxième unité d'entraînement (162) est un dispositif de déplacement biaxial (670B), qui comporte un support (671B) destiné à porter respectivement le premier ou le deuxième élément de film optique réfléchissant (140B), comporte un mécanisme de fourniture d'énergie mécanique (672B) couplé au support (671B) et entraînant le support (671B) pour qu'il se déplace dans une première direction axiale (D1), de manière à entraîner l'élément de film optique réfléchissant (140B) pour qu'il se déplace dans la première direction axiale (D1), comporte un autre support (673B) destiné à supporter le mécanisme de fourniture d'énergie mécanique (672B), et comporte un autre mécanisme d'entraînement mécanique (674B) couplé à l'autre support (673B) et entraînant l'autre support (673B) à se déplacer dans une deuxième direction axiale (D2) différente de la première direction axiale (D1), de manière à entraîner l'élément de film optique réfléchissant (140B) à se déplacer dans la deuxième direction axiale (D2), de manière à permettre au premier ou au deuxième élément de film optique réfléchissant (140B) respectif de se déplacer le long de la première direction axiale (D1) et/ou de la deuxième direction axiale (D2), de sorte que l'une des unités de filtre réfléchissant (FU, FU1, FU2, FU3, FU4) du premier élément de film optique réfléchissant recoupe un trajet de transmission du faisceau d'excitation (ELo), et/ou l'une des unités de filtre réfléchissant (FU, FU1, FU2, FU3, FU4) du deuxième élément de film optique réfléchissant recoupe un trajet de transmission du faisceau de fluorescence (FLo),

ou

2) la première unité d'entraînement (161) et/ou la deuxième unité d'entraînement (162) est un dispositif de plateau tournant mobile (670C), qui comporte un support (671C) destiné à porter le premier ou le deuxième élément de film optique réfléchissant respectif (140C), comporte un mécanisme de fourniture d'énergie mécanique (672C) couplé au support (671C) et entraînant le support (671C) en rotation par rapport à une troisième direction axiale (D3), de manière à entraîner le premier ou le deuxième élément de film optique réfléchissant (140B) en rotation par rapport à la troisième direction axiale (D3), comporte un autre support (673C) destiné à supporter le mécanisme de fourniture d'énergie mécanique (672C), et comporte un autre mécanisme d'entraînement mécanique (674C) couplé à l'autre support (673C) et entraînant l'autre support (673C) à se déplacer dans la première direction axiale (D1), de manière à entraîner le premier ou le deuxième élément de film optique réfléchissant (140B) à se déplacer dans la première direction axiale (D1), de sorte que les multiples unités de filtre réfléchissant (FU) du premier ou du deuxième élément de film optique réfléchissant respectif (140C) puissent être respectivement situées à des positions différentes dans la direction radiale ou la direction circonférentielle du dispositif de plateau tournant mobile (670C) de sorte que, lorsque le dispositif de plateau tournant mobile (670C) tourne ou se déplace, l'une des unités de filtre réfléchissant (FU, FU1, FU2, FU3, FU4) du premier élément de film optique réfléchissant intercepte un trajet de transmission du faisceau d'excitation (ELo), et/ou l'une des unités de filtre réfléchissant (FU, FU1, FU2, FU3, FU4) du deuxième élément de film optique réfléchissant intercepte un trajet de transmission du faisceau de fluorescence (FLo).

2. L'utilisation d'un système de réaction en chaîne par polymérase quantitative (qPCR) (10) selon la revendication 1 dans un procédé de détection destiné à surveiller, enregistrer et analyser quantitativement et/ou qualitativement en temps réel le changement de température et le changement de fluorescence d'un échantillon testé au cours d'un processus de réaction en chaîne par polymérase sous le contrôle de l'unité de commande (150), le procédé de détection comprenant :
la commande, à l'aide de l'unité de commande (150), de la première unité d'entraînement (161) et/ou de la deuxième unité d'entraînement pour effectuer l'une des étapes suivantes 1) ou 2) :

1) entraîner le support (671B) à se déplacer dans la première direction axiale (D1), de manière à entraîner l'élément de film optique réfléchissant (140B) à se déplacer dans la première direction axiale (D1), et entraîner l'autre support (673B) à se déplacer dans la deuxième direction axiale (D2) différente de la première direction axiale (D1), de manière à entraîner l'élément de film optique réfléchissant (140B) à se déplacer dans la deuxième direction axiale (D2), de manière à permettre au premier ou au deuxième élément de film optique réfléchissant (140B) respectif de se déplacer le long de la première direction axiale (D1) et/ou de la deuxième direction axiale (D2), de sorte que l'une des unités de filtre réfléchissant (FU, FU1, FU2, FU3, FU4) du premier élément de film optique réfléchissant intercepte un trajet de transmission du faisceau d'excitation (ELo), et/ou l'une des unités de filtre réfléchissant (FU, FU1, FU2, FU3, FU4) du deuxième élément de film optique réfléchissant intercepte un trajet de transmission du faisceau de fluorescence (FLo) ; ou
2) entraîner le support (671C) à tourner par rapport à une troisième direction axiale (D3), de manière à entraîner le premier ou le deuxième élément de film optique réfléchissant (140B) respectif à tourner par rapport à la troisième direction axiale (D3), et entraîner l'autre support (673C) à se déplacer dans la première direction axiale (D1), de manière à entraîner le premier ou le deuxième élément de film optique réfléchissant (140B) respectif à se déplacer dans la première direction axiale (D1), de sorte que les multiples unités de filtre réfléchissant (FU) du premier ou du deuxième élément de film optique réfléchissant (140C) puissent être respectivement situées à des positions différentes dans la direction radiale ou la direction circonférentielle du dispositif de plateau tournant mobile (670C) de sorte que, lorsque le dispositif de plateau tournant mobile (670C) tourne ou se déplace, l'une des unités de filtre réfléchissant (FU, FU1, FU2, FU3, FU4) du premier élément de film optique réfléchissant intercepte un trajet de transmission du faisceau d'excitation (ELo), et/ou l'une des unités de filtre réfléchissant (FU, FU1, FU2, FU3, FU4) du deuxième élément de film optique réfléchissant intercepte un trajet de transmission du faisceau de fluorescence (FLo).

FIG. 1

FIG. 2

FIG. 3A

IL(ELi,FLi)  OL1  OL2

$OL(ELo,FLo) \begin{cases} OL1 \\ OL2 \end{cases}$

S1

S2

Tx

## FIG. 3B

IL(ELi,FLi)  OL1  OL2

$OL(ELo,FLo) \begin{cases} OL1 \\ OL2 \end{cases}$

$\theta 0$

S1

$\theta x$

$\theta x$

S2

Tx

## FIG. 3C

EP 4 006 528 B1

FIG. 3D

25

FIG. 3E

Turn on the light emitting element — S110

Control the reflective filter unit of the first reflective optical film element to cut into the transmission path of the excitation beam according to the excitation wavelength range, so that the test specimen receives the excitation beam whose main emission wavelength falls within the excitation wavelength range to generate the fluorescence beam — S120

Control the reflective filter unit of the second reflective optical film element to cut into the transmission path of the fluorescence beam according to the detection wavelength range to purify the characteristics of the fluorescence beam — S130

The light detection element detects the light intensity of the fluorescence beam — S140

FIG. 4

FIG. 5A

FIG. 5B

FIG. 5C

FIG. 5D

FIG. 6A

EP 4 006 528 B1

FIG. 6B

**FIG. 6C**

EP 4 006 528 B1

FIG. 7

EP 4 006 528 B1

FIG. 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 397496 A1
- US 20050151972 A1
- US 2016230210 A1